(19) **Europäisches Patentamt / European Patent Office / Office européen des brevets**

(11) **EP 2 087 903 A1**

(12) # EUROPEAN PATENT APPLICATION
published in accordance with Art. 153(4) EPC

(43) Date of publication:
**12.08.2009 Bulletin 2009/33**

(21) Application number: **07832357.3**

(22) Date of filing: **22.11.2007**

(51) Int Cl.:
*A61K 36/70* (2006.01)   *A23K 1/16* (2006.01)
*A23L 1/30* (2006.01)   *A61K 8/73* (2006.01)
*A61K 8/97* (2006.01)   *A61K 47/36* (2006.01)
*A61P 1/00* (2006.01)   *A61P 1/02* (2006.01)
*A61P 1/16* (2006.01)   *A61P 1/18* (2006.01)
*A61P 3/04* (2006.01)   *A61P 3/06* (2006.01)
*A61P 3/10* (2006.01)   *A61P 9/00* (2006.01)
*A61P 9/10* (2006.01)   *A61P 11/00* (2006.01)
*A61P 13/12* (2006.01)   *A61P 17/00* (2006.01)
*A61P 17/14* (2006.01)   *A61P 17/16* (2006.01)
*A61P 17/18* (2006.01)

(86) International application number:
**PCT/JP2007/072628**

(87) International publication number:
**WO 2008/062861 (29.05.2008 Gazette 2008/22)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **24.11.2006   JP 2006317818**

(71) Applicant: **Kabushiki Kaisha Hayashibara Seibutsu Kagaku Kenkyujo Okayama-shi Okayama 700-0907 (JP)**

(72) Inventors:
 • **FUJII, Mitsukiyo**
   **Okayama-shi**
   **Okayama 700-0907 (JP)**
 • **USHIO, Shimpei**
   **Okayama-shi**
   **Okayama 700-0907 (JP)**
 • **IWAKI, Kanso**
   **Okayama-shi**
   **Okayama 700-0907 (JP)**
 • **KYONO, Fumiyo**
   **Okayama-shi**
   **Okayama 700-0907 (JP)**

(74) Representative: **Daniels, Jeffrey Nicholas**
   **Page White & Farrer**
   **Bedford House**
   **John Street**
   **London WC1N 2BF (GB)**

(54) **EXTRACT POWDER OF INDIGO-CONTAINING PLANT, METHOD FOR PRODUCTION THEREOF, AND USE THEREOF**

(57)   The present invention has objects to provide an extract powder of indigo plant, which has a relatively low hygroscopicity, satisfactory fluidability, and improved storage stability; a process thereof; and a composition incorporated with the extract powder. The objects are solved by a process for producing an extract powder of indigo plant, which comprises incorporating a partial starch hydrolyzate with a dextrose equivalent of 10.2 or lower into one part by weight, on a dry solid basis, of an indigo plant extract contained in a liquid extract of indigo plant in an amount of not lower than 0.25 part by weight but not higher than 5 parts by weight, on a dry solid basis, and drying the resulting mixture; an extract powder of indigo plant prepared thereby; and a composition incorporated with the extract powder.

EP 2 087 903 A1

**Description**

Technical Field

The present invention relates to an extract powder of indigo plant, its preparation and uses, particularly, to a powder of an indigo plant extracted with a solvent containing water, and its preparation and uses.

Background Art

Indigo plants are called indigo-containing plants which contain indican, capable of forming indigo used for preparing dyes for dying with indigo; examples of such are *Polygonum tinctorium* Lour., *Strobilanthes cusia* Nees. (a plant of the family *Justicia*), *Isatis tinctoria L. var yezoensis* (Ford; a plant of the family *Cruciferae*), *Mercurialis leiocarpa* Sieb. (a plant of the family *Euphorbiaceae*), and *Indigofera suffruticosa* (a plant of the family *Leguminosae*). In Japan, *Polygonum tinctorium* Lour. has been used not only in preparing dyes but used as quasi-drugs from ancient times because indigo leaves and fruits have been recognized to contain useful physiologically active ingredients and therefore dried leaves and fruits of *Polygonum tinctorium* Lour. have been used for such purposes. Recently, there have been provided food products, cosmetics, pharmaceuticals, etc., which contain indigo plant extracts, expecting their anti-tumor, anti-viral, antibacterial, and allergic activities (see, for example, Japanese Patent Kokai Nos. 2001-31581 and 2004-189732 and International Patent Publication No. WO 2003/035091).

Liquid extracts of indigo plants, particularly, those prepared with aqueous solvents have been used in external dermal agents and food products, however, when stored intact, they may be spoiled in their stability or solubility, or may induce sediments, smell, or color, resulting in difficulty of being incorporated into white cosmetic-creams. Since such liquid extracts are susceptible to form sediments or precipitates as the increase of concentration of solid contents, the increment of level of the liquid extracts to be added to compositions may become difficult in some cases. It was revealed that, considering easiness of handleability and improvement of the level of such increment, when the liquid extracts are merely freeze-dried or sprayed intact, there could only be obtained unstable powders that are strongly hygroscopic just after their preparations and then easily solidified, and it may cause problems of, for example, progressive browning during storage. Although powders from liquid extracts of indigo plants with sugar alcohols such as maltitol or with oyster shells are commercialized, they have a relatively high hygroscopicity and an extremely low content of an indigo plant extract in a ratio of 1:50 by weight in terms of a filler to the indigo plant extract, on a dry solid basis (d.s.b.), and this would restrict the ratio of the indigo plant extract external dermal agents and food products, when preparing such compositions. In preparing external dermal agents and orally ingestible products such as food products, which are incorporated with an indigo plant extract, it is highly desired a supplying of extract powders of indigo plants, which have an improved content of the indigo plant extracts, readily handleability, and storage stability, however, no such preparation method thereof has not yet been established.

Disclosure of Invention

The present invention has objects to provide an extract powder of indigo plant, which is rich in indigo plant extract, readily handleable, scarcely hygroscopic, and relatively high in storage stability; and its preparation and uses.
To solve the above objects, the present inventors energetically studied the preparation method of such an extract powder of indigo plant. As a result, they found that the desired powder, which is rich in indigo plant extract and high in storage stability, can be easily produced by incorporating a partial starch hydrolyzate with a dextrose equivalent (abbreviated as "DE", hereinafter) of 10.2 or lower in an amount of 0.25 part by weight or higher but not higher than 5 parts by weight, d.s.b., to one part by weight, d.s.b., of a solid of liquid extract of indigo plant dissolved and/or suspended in any of the later described aqueous solvents, drying the resulting mixture, and optionally pulverizing the dried product. Further, the present inventors found that external dermal agents such as cosmetics, quasi-drugs, and pharmaceuticals; orally ingestible compositions such as food products, pharmaceuticals, and quasi-drugs; feeds; baits; pet foods; and miscellaneous goods can be easily prepared by using the extract powder of indigo plant of the present invention. Thus, they accomplished this invention.
The present invention solves the above objects by providing a preparation method comprising the steps of incorporating a partial starch hydrolysate with a DE of 10.2 or lower in an amount of 0.25 to 5 parts by weight, d.s.b., to one part by weight, d.s.b., of the solid of a liquid extract of indigo plant, and drying the resulting mixture; an extract powder of indigo plant produced by the method; and a composition incorporated with the extract powder.
The extract powder of indigo plant of the present invention has a relatively high safeness, insubstantial hygroscopicity and browning, and improved storage stability. Since the extract powder of indigo plant has a melanin-formation inhibitory activity, etc., it exhibits a satisfactory skin-whitening effect of lightening the skin color of post sunburn pigmentation, spots, freckles, and chloasmata, when applied extradermally or ingested orally. Also, the extract powder of indigo plant has an elastase inhibitory activity, turnover-accelerating activity of skin cells, etc., and this inhibits the formation of wrinkles and fine wrinkles and recovers/maintains the tension and elasticity of the skin, resulting in exertion of satisfactory effects of the prevention of skin ageing and the maintenance of youthful skin conditions. The extract powder of indigo plant has various physiological actions such as moisture-retaining action to the skin, lipase-activity-inhibitory action, action of inhibiting secretion of sebums from sebaceous glands, action of promoting decomposition of body fats, antibacterial action, antioxidant action, anti-inflammatory action, anti-periodontal action, action of improving lipid metabolism, action of lowering body fats, etc.

By preparing external dermal agents and orally ingestible compositions using the extract powder of indigo plant of the present invention, the physiological activities of the indigo plant can be imparted thereto.

Brief Description of Drawings

FIG. 1 is a graph which shows the relationship between the concentration of the indigo extract in the extract powder of indigo plant of the present invention and the inhibitory percentage of elastase activity.

FIG. 2 is a graph which shows the relationship between the concentration of the indigo extract in the extract powder of indigo plant of the present invention and the inhibitory percentage of lipase activity.

FIG. 3 is a graph which shows the relationship between the concentration of the indigo extract in the extract powder of indigo plant of the present invention and the elimination percentage of $O^{2-}$.

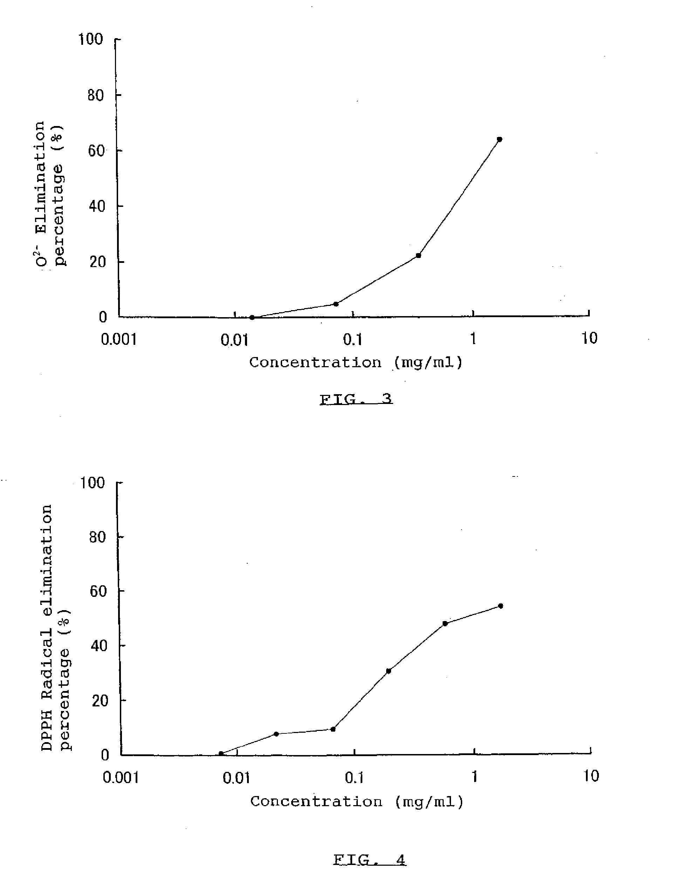

FIG. 3 is a graph which shows the relationship between the concentration of the indigo extract in the extract powder of indigo plant of the present invention and the elimination percentage of DPPH radical.

Best Mode for Carrying Out the Invention

The term "indigo plant(s)" as referred to as in the present invention means so called indigo-containing plants which contain indican that plays a major role in preparing dyes for dying with indigo; concrete examples of such are *Polygonum tinctorium* Lour. (a plant of the family *Polygonaceae*), *Strobilanthes cusia* Nees. (a plant of the family *Justicia*), *Isatis tinctoria* L. *var yezoensis* (Ford.; a plant of the family *Cruciferae*), *Mercurialis* leiocarpa Sieb. *et* Zucc. (a plant of the family *Euphorbiaceae*), *Indigofera suffruticosa* (a plant of the family *Leguminosae*), as well as other plants of the families *Compositae, Oleaceae* and *Orchidaceae* (may be called "indigo plant(s)" as a general term throughout the specification, hereinafter). Among which *Polygonum tinctorium* Lour, an annual plant of the family *Polygonaceae,* is desirable for use on an industrial-scale production because it is readily available and rich in tryptanthrin as a characteristic ingredient thereof.

The indigo plants usable in the present invention should not specifically be restricted to their origins and cultures, any of those which grow naturally and agriculturally, as well as mutants obtainable by culturing in conventional manner and other cultures obtainable through tissue culture, callus culture, cell culture, etc., can be used. In the case of using plant bodies as materials for extracts, a part or the whole of the plant bodies can be used. These materials can be in any form of a fresh preparation, i.e., those in a condition of a humid form, freeze-dried form, or mixture form thereof. From a point of view of handleability, dried ones are preferable.

Examples of the method for preparing the extract powder of indigo plant of the present invention include those which contain the steps of providing a liquid extract of indigo plant prepared from a part and/or the whole of the indigo plant body as indicated above with or without optional concentration in usual manner, depending on use; incorporating a partial starch hydrolyzate with a DE of 10.2 or lower as a pulverizing base in an amount of at least 0.25 but not more than 5 parts by weight, d.s.b., preferably, at least 0.5 part by weight but not more than 2.5 parts by weight, d.s.b., to one part by weight, d.s.b., of the solid contents of the liquid extract; drying the resulting mixture and optionally pulverizing the dried product to obtain a stable extract powder of indigo plant. Referring to the amount of the above-identified partial starch hydrolyzate with a DE of 10.2 or lower, the more the amount to the indigo plant extract, the more the stability of the resulting powder is improved. Use of an excessive amount of such partial starch hydrolyzate will increase both the viscosity of the above mixture and the energy cost for drying and will reduce the content of indigo plant extract in the resulting powder, while an increment of the powder in an objective composition will increase an opportunity of deteriorating the property of such composition and therefore the content of indigo plant extract in the powder should preferably be as high as possible.

Examples of the partial starch hydrolyzate preferably used as the base for pulverization include those which have a relatively low decomposition rate, i.e., those with a DE of 10.2 or lower, preferably, a DE of not lower than 0.2 but not higher than 5.2, more preferably, a DE of not lower than 0.2 but not higher than 4.1. Preferred starches usable as materials include cereal powders of starches, as a main ingredient, such as corn starch powders, wheat flours, and rice powders, as well as starches from these cereals; and further include tuber powders such as sweet potato powders, potato powders, and tapioca powders, as well as starches from these tubers. Examples of the method for preparing partial starch hydrolyzates include, for example, those which contain the steps of partially decomposing the above-identified starches with acids and neutralizing the resulting mixtures; or partially hydrolyzing such starches with α-amylase or cyclomatodextrin glucanotransferase up to give a decomposition degree of a DE of 10.2 or lower, preferably, a DE of 8 or lower, and then optionally subjecting the mixtures to filtration, decoloration, desalination, and concentration in usual manner and then optionally pulverizing the resulting mixtures. Commercialized dextrins, cyclodextrins, and partial starch hydrolyzates containing cyclodextrins, which have a DE of 10.2 or lower, can be advantageously used.

Among the partial starch hydrolyzates usable in the present invention, examples of the non-reducing oligosaccharides include α,α-trehalose, saccharide derivatives of α,α-trehalose, a cyclic tetrasaccharide (cyclonigerosylnigerose) having the structure of cyclo{→6)-α-D-glucopyranosyl-(1→3)-α-D-glucopyranosyl-(1→6)-α-D-glucopyranosyl-(1→3)-α-D-glucopyranosyl-(1→} disclosed in International Patent Publication No. WO 02/10361, a cyclic tetrasaccharide (cyclomaltosylmaltose) having the structure of cyclo{→6)-α-D-glucopyranosyl-(1→4)-α-D-glucopyranosyl-(1→6)-α-D-glucopyranosyl-(1→4)-α-D-glucopyranosyl-(1→} disclosed in Japanese Patent Kokai No. 2005-95148, and cyclic penta- or hexa-saccharide having the structure of cyclo{→6)-[α-D-glucopyranosyl-(1→4)]n-α-D-glucopyranosyl-(1→} (n means 4 or 5) disclosed in International Patent Application No. PCT/JP2005/17642, and derivatives of these cyclic oligosaccharides. Since bases for pulverization rich in sugar alcohols such as sorbitol and maltitol are susceptible to cause hygroscopicity, the content of these sugar alcohols should preferably be as low as possible.

The method for drying the liquid extract of indigo plant should not specifically be restricted and any of freeze drying, spray drying, drum drying methods, etc., can be used. Among which freeze-drying method and spray-drying method, particularly, spray-drying method is preferable because it can prepare the desired powder in a relatively large amount and at a lesser cost. Examples of such spray-drying method include various ones of conventional rotating-disc method, pressure-nozzle method, two-fluid-nozzle method, etc., which can be appropriately selected. Pulverization method for the resulting dried product should not specifically be restricted and any conventional ones can be employed.

The extract powder of indigo plant thus obtained is a powder which usually has a water content of about 2 to 5% and has insubstantial hygroscopicity, satisfactory floatability, and improved storage stability. The extract powder can be used intact, volumed up with an additional filler such as saccharides, or admixed with one or more ingredients usable for cosmetics, quasi-drugs, pharmaceuticals, food products, feeds, baits, pet foods, and miscellaneous goods before use in various compositions such as external dermal agents, orally ingestible products, and intubationally administrable products. The extract powder of indigo plant of the present invention can be added to the desired compositions before or after completion of their processings, and concrete examples of methods for such purpose are mixing, kneading, dissolving, melting, spreading, suspending, emulsifying, penetrating, crystallizing, sprinkling, applying, adhering, spraying, coating, injecting, soaking, and solidifying, one or more of which can be selected in an appropriate combination.

The liquid extract of indigo plant usable in the present invention can be prepared by adding water or a water-containing solvent to a part or the whole of an indigo plant body and subjecting the resulting mixture to any of conventional extraction methods in general. In addition to the above method, the liquid extract can be prepared by using juices of indigo plant, mixing the liquid extract with such juices, or by using liquid extracts of indigo plant extracted with hydrophobic organic solvents. Before extraction with solvents, the above-identified materials can be previously treated with physical and/or chemical treatments of one or more of cutting, crushing, frictional crushing, pulverizing, expressing, fermenting, drying, and freezing to make into cut-, crushed-, fictionally crushed-, pulverized-, expressed-, fermented-, dried-, and freeze dried-materials of indigo plants, one or more of which can be appropriately used as materials for extraction. When powdering the above materials of indigo plants with extraction solvents of hydrophobic organic solvents, they can be mixed with water and an optional emulsifier to be suspended and/or dissolved therein, stirred and mixed with partial starch hydrolyzates, and then dried by the above-identified drying methods.

Examples of the solvents usable for preparing liquid extracts of indigo plants used in preparing the extract powder of indigo plant of the present invention include aqueous solvents (for example, water and acid or basic aqueous solvents), alcohols (for example, lower alcohols such as methanol, anhydrous ethanol, and ethanol; polyhydric alcohols such as propylene glycol and 1,3-butylene glycol (abbreviated as "1,3-GB", hereinafter); and ketones such as acetone, one or more of which can be mixed in an appropriate combination before use. Among the solvents, in view of the intensity and safeness of skin-whitening action and skin-beautifying action, water is preferable and solvent mixtures containing water are also desirable (throughout the specification, liquid extracts of indigo plants extracted with water or water-containing solvents may be generally called "liquid extracts of indigo plants"). As the solvent mixtures, a most preferable one is a combination of water and ethanol, usually, aqueous ethanol solutions with ethanol concentrations of 0 to 70% by weight (throughout the specification, "% by weight" is designated as "%" unless specified otherwise, hereinafter) are preferable, and those with ethanol concentrations of 0 to 60% by weight are particularly preferable. The extraction solvents can be arbitrarily controlled to the desired pHs with appropriate acids, alkalis, or buffers. The extraction can be conducted usually at a pH in the range of 2 to 13, preferably, in the range of 4 to 10, and most preferably, in the range of 4 to 7.5.

To prepare the liquid extract of indigo plant, an appropriate amount of any one of the above-identified extraction solvents can be used with any of the above-identified materials, usually, the former is added to the later in an amount of 0.1 to 30 times, desirably, 0.5 to 2 times by weight of the later, and optionally the materials are extracted with treatments of stirring, heating, pressing, ultrasonication, etc.; followed by separating the resulting extracts into a liquid part and a residual part by applying appropriate methods such as filtration, centrifugation, and decantation; and collecting the liquid part to obtain a desired liquid extract of indigo plant. The residue part can be further repeatedly treated with similar treatment as used in the above, and the liquid parts respectively collected in each treatment can be arbitrarily pooled into an extract. In the case of treating these materials and residues with extraction treatments twice or more, they can be independently treated with different extraction solvents to collect the desired liquid parts or they can be arbitrarily pooled into a liquid extract. The extraction step with solvents should not specifically be restricted as long as it gives a time sufficient to extract the desired ingredients, usually, the desired extraction time is about one minute to about 120 hours, preferably, about five minutes to about 48 hours, varying depending on the pulverization degree of indigo plants or the temperature of extraction solvents used.

In preparing these liquid extracts of indigo plants, the later described one or more of surfactants, antiseptics (antibacterial agents), humectants, viscosity-imparting agents, water-soluble high molecules, antioxidants, chelating agents, dyes/pigments, flavors, pH-controlling agents, vitamins such as L-ascorbic acid and derivatives thereof, and additives can be added to the liquid extracts for the purpose of inhibiting the formation of grouts and browning, the generation of smell, and the proliferation of microorganisms. As long as it does not inhibit the effects and functions of the present invention, the timing and the amount of the above ingredients to be added should not specifically be restricted and the ingredients can be added at an appropriate timing during the processings of the liquid extracts of indigo extracts.

The liquid extracts of indigo plants thus obtained can be arbitrarily pulverized intact or after treated with appropriate purification treatments for improving the physiological activities of indigo plants to make into liquid extracts of indigo plants with improved contents of their effective ingredients. Depending on use, the liquid extracts can be purified to increase or decrease the percentages of one or more of components of polyphenols, alkaloids, terpenoids, steroids, phenols, dyes/ pigments, saccharides, proteins, amino acids, nucleic acids, peptides, lipids, etc.; and then pulverized. Varying depending on the types of the objective ingredients, purification techniques are appropriately selected from, for example, filtration, concentration, centrifugal separation, separation with solvents, separatory sedimentation, dialysis, hydrophobic chromatography, reverse-phase chromatography, adsorption chromatography, affinity chromatography, gel filtration chromatography, and/or ion-exchange chromatography. Treatments with ultrafiltration and micro-filtration can be used to remove solids of grouts and high molecular fractions, which are causatives of grout formation or allergy, without reducing the effective ingredients of indigo plants, and used to obtain clear liquid extracts for preparing the extract powder of indigo plant of the present invention. Considering the level of physiological activity of skin-whitening activity, skin-beautifying activity, retrogradation-preventing activity, anti-periodontal disease, action of lowering body fats, the liquid extract of indigo plant according to the present invention should preferably contain at least 200 $\mu$g/ml of polyphenols and at least 0.5 $\mu$g/ml of tryptanthrin, and most preferably, at least 400 $\mu$g/ml of polyphenols and at least 1.0 $\mu$g/ml of tryptanthrin. The liquid extracts of indigo plants can be advantageously pulverized after increasing the contents of the effective ingredients such as tryptanthrin, gallic acid, caffeic acid, kaempferol, etc., which are representative ingredients of the liquid extract that play an important role on skin-whitening action, anti-ageing action, antiseptic action, anti-inflammatory action, etc., inherent to the extracts.

These liquid extracts of indigo plants can be optionally treated with centrifugation, filtration such as ultrafiltration and micro-filtration, or concentration; admixed with at least one of physiologically acceptable ingredients or others which can be appropriately incorporated into foods, cosmetics, quasi-drugs, pharmaceuticals, feeds, baits, pet foods, etc.; and pulverized. To concentrate the liquid extracts of indigo plants, conventional methods used in the fields of food, cosmetic, pharmaceutical industries, etc., for example, methods such as concentration *in vacuo* and concentration with a reverse osmotic membrane can be appropriately used in combination.

The extract powder of indigo plant of the present invention has not only a melanin formation inhibitory action, elastase activity inhibitory action, anti-ageing action, lipase activity inhibitory action, and action of inhibiting secretion of sebums from sebaceous glands, but has at least the following actions:

(1) It entraps *in vivo* radicals derived from active oxygen and lipid peroxide which are recognized to be causatives of diseases such as malignant tumors, myocardial infarction, stroke, rheumatoid, life-style-related diseases (geriatric diseases), renal disorders, and hemorrhoids; stresses; and ageing;

(2) It regulates the production of cytokines, including interferon-$\gamma$ and interleukin-10, by immunocompetent cells relating to the decision of *in vivo* balance between type 1 helper T-cells (Th1) and type 2 helper T-cells (Th2), and controls the *in vivo* balance to its intrinsic normal conditions, and treats/prevents diseases such as autoimmune disease, hepatopathy, renal disorders, pancreatic disorders, and graft versus host diseases (GVHD), which are inducible by abnormality of the balance; and

(3) It inhibits both the expression of nitric oxide synthase in *in vivo* cells induced by, for example, stimulations of ultraviolet ray, cytokines or endotoxins, and the activity of prostaglandin synthase. Through the inhibition of the formation of nitric oxide and prostaglandin $E_2$ by the action of the above enzymes, the melanogenesis in melanocytes inducible by the stimulations of the above substances is inhibited to exert skin-beautifying action. It also treats/prevents diseases such as autoimmune diseases, allergic diseases, inflammatory diseases, malignant tumors, renal disorders, and lung disorders, relating to nitrogen oxide and prostaglandin $E_2$ over expressed *in vivo*.

The extract powder of indigo plant exerts properties of controlling biological functions and acts on disorders of biological tissues that accompany inflammation inducible by the infection of microorganisms such as gram-positive bacteria, gram-negative bacteria, fungi, and viruses; the invasion of or contact with foreign substances to living bodies such as proteins, organic compounds, and metals; or by the generation of tumors so as to regulate such inflammation through the inhibition of the production of inflammatory cytokines including tumor necrosis factor (TNF), interferon-γ, and interleukin-1.

Since the extract powder of indigo plant exerts a relatively strong bacteriostatic and/or germicidal action against bacteria which are causatives of periodontal diseases including *Prophyromonas gingivalis* (may be abbreviated as "*P. gingivalis*", hereinafter) residing on the tooth surfaces or pockets, and dental-caries-inducing bacteria such as *Streptococcus mutans* (may be abbreviated as "*S. mutans*", hereinafter), it inhibits swelling and inflammation of gums and necks, and intraoral bleeding, controls intraoral plaques, and prevents/improves periodontal diseases and dental caries, when used in intraoral external agents and orally ingestible compositions. Because of these, the extract powder lowers the onset risk of aspiration pneumoniae and pneumoniae that is inducible by intraoral bacteria, as well as diseases inducible or accompanied by periodontal diseases, such as nephritis, sepsis, bacteriemia, bacterial pericarditis, arterial diseases, birth of low-weight baby, diabetes, and esophageal carcinoma. Therefore, the extract powder of indigo plant can be advantageously used as melanin-formation inhibitory agents, elastase activity inhibitory agents, anti-ageing agents, lipase activity inhibitory agents, and/or agents for regulating the secretion of sebums from sebaceous glands; agents for exerting antibacterial action against microorganisms including those which are causative of dermatophytosis and acne, anti-inflammatory action on dermatological diseases such as stomatitis, dermatitis, eczema, and eruption, anti-ageing action, anti-periodontal disease action, anti-dental-caries action, active-oxygen-eliminating action, radical-entrapping action, production-regulatory and production-inhibitory actions of cytokines, expression inhibitory action of nitrogen oxide synthase, activity inhibitory action of prostaglandin synthase, and/or medicines for moderately exerting biological function regulatory action; external dermal agents such as cosmetics, pharmaceuticals, and quasi-drugs; and others such as food products, feeds, baits, pet foods, and miscellaneous goods.

Since the extract powder of indigo plant of the present invention has an improved effect of promoting the growth of papilla pili cells, it can be used as an effective ingredient of hair restorers and orally ingestible compositions, and used to improve the survival rate of hair roots by successively cutting out the hair roots from the scalp and treating them with a soaking solution dissolving the extract powder, when the hair roots are implanted. The extract powder of indigo plant of the present invention inhibits the increase of adipocytes and expedites the metabolism/decomposition and the burning of body fats to effectively lower skinfold thickness, and thus it can be incorporated into orally ingestible compositions and efficiently used in the prevention and treatment of life-style-related diseases and metabolic syndromes including obesity, diabetes, hypertension, and hyperlipemia; and also it can be incorporated into external dermal agents as slimming agents to reduce systemic or local fats.

The amount of the extract powder of indigo plant of the present invention to be incorporated into the above-identified compositions should not specifically be restricted as long as it exerts the desired effects and functions inherent to an indigo extract, for example, it is usually 0.0005 to 30%, preferably, 0.005 to 10%, most preferably, 0.05 to 10% of the indigo extract, d.s.b., to the total amount of each composition. The desired effects and functions could not be attained at a lesser concentration of 0.0005%, and the levels could not be increased even when the extract powder of indigo plant is used in an amount of 30% or higher, and the properties of the compositions may be affected depending on the forms of the compositions.

Now explaining the case of incorporating the extract powder of indigo plant of the present invention into external dermal agents such as cosmetics, pharmaceuticals, and quasi-drugs, the extract powder can be incorporated alone or in combination with one or more other ingredients with skin-whitening action to synergistically enhance the melanin-formation inhibitory action, elastase activity inhibitory action, lipase activity inhibitory action, and/or anti-ageing action, which are inherent to the extract powder, but also enhance the well-known physiological activities such as antioxidant action, anti-inflammatory action, and antiseptic action, which are inherent to indigo extracts. As the other ingredients with skin-whitening action other than the extract powder of indigo plant, any ingredients can be used as long as they enhance the physiological activities of the extract powder of indigo plant without restriction of their origins and preparation methods. For example, as concrete examples of such, L-ascorbic acid and their derivatives and salts; alkoxy salicylic acids and salts thereof; hydroquinone glycosides and derivatives thereof, tranexamic acid and their derivatives and salts, resorcin derivatives; kojic acid and their derivatives and salts; ellagic acid, linoleic acid, and their salts; chamomile extract; tetrahydrocurcuminoid; and vitamin P such as hesperidin and derivatives thereof. Considering the strength of enhancing the effect of these physiological activities inherent to the extract powder of indigo plant, ascorbic acid and derivatives thereof are preferable, and L-ascorbic acid 2-glucoside is particularly preferable.

The amount of one or more of the other ingredients with skin-whitening action used in combination with the extract powder of indigo plant should not specifically be restricted as long as it has no pharmaceutical problem, it is usually an amount of 0.001 to 20% by weight to the total amount of an external dermal agent. When the amount of less than 0.001%, the enhancement effect on the skin-whitening effect of an external dermal agent tends to become poor, while, even when incorporated in an amount of over 20%, any increased effect could not substantially be expected and the incorporation into an external dermal agent tends to become harder. More preferable amount is in the range of 0.01 to 10% and most preferable one is in the range of 0.1 to 10%. In the case of a chamomile extract, the amount is meant in terms of its dry solid basis.

The L-ascorbic acid and derivatives thereof usable in the present invention should not specifically be restricted. Concrete examples of such include L-ascorbic acid and derivatives thereof including L-ascorbic acid alkyl esters such as L-ascorbic acid monostearate, L-ascorbic acid monopalmitate, L-ascorbic acid monooleate, L-ascorbic acid distearate, L-ascorbic acid dipalmitate, and L-ascorbic acid dioleate; L-ascorbic acid phosphates such as L-ascorbic acid phosphate ester, magnesium L-ascorbic acid phosphate ester, and sodium L-ascorbic acid phosphate ester, as well as salts thereof; L-ascorbic acid sulfates and salts thereof; L-ascorbic acid 2-glycosides including L-ascorbic acid 2-glucoside as a preferable example; and acyl derivatives thereof. In addition to alkaline metal salts and alkaline earth metal salts of L-ascorbic acid such as of sodium salts, potassium salts, magnesium salts, and calcium salts, and other salts of L-ascorbic acid such as of ammonium salts and amino acid salts can be similarly used as the above. In the case of salts of L-ascorbic acid phosphates, those of alkaline earth metals are preferable and those of magnesium salts are more preferable. In view of the stability when used in external dermal agents, preferable derivatives of L-ascorbic acid usable in the present invention include one or more members selected from L-ascorbic acid alkyl esters, L-ascorbic acid phosphates, L-ascorbic acid, and salts thereof; L-ascorbic acid sulfates and salts thereof; and L-ascorbic acid 2-glucoside and salts thereof.

The alkoxy salicylic acids usable in the present invention mean derivatives of salicylic acid where any of the hydrogen atoms of C-3, C-4 or C-5 position has been substituted with an alkoxy group. Preferable examples of the alkoxy group include methoxy group, ethoxy group, propoxy group, isopropoxy group, butoxy group, isobutoxy group, etc. Among which, methoxy group and ethoxy group are particularly preferable.

Concrete examples of the alkoxy salicylic acids include 3-methoxysalicylic acid (2-hydroxy-3-methoxybenzoic acid), 3-ethoxysalicylic acid (2-hydroxy-3-ethoxybenzoic acid), 4-methoxysalicylic acid (2-hydroxy-4-methoxybenzoic acid), 4-ethoxysalicylic acid (2-hydroxy-4-ethoxybenzoic acid), 4-propoxysalicylic acid (2-hydroxy-4-propoxybenzoic acid), 4-isopropoxysalicylic acid (2-hydroxy-4-isopropoxybenzoic acid), 4-buthoxysalicylic acid (2-hydroxy-4-butoxybenzoic acid), 5-methoxysalicylic acid (2-hydroxy-5-methoxybenzoic acid), 5-ethoxysalicylic acid (2-hydroxy-5-ethoxybenzoic acid), 5-propoxysalicylic acid (2-hydroxy-5-propoxybenzoic acid), etc. In view of the level of skin-whitening effect, 4-methoxysalicylic acid is particularly preferable.

Alkoxysalicylic acid in the form of a salt can be incorporated into the external dermal agent of the present invention. Preferable examples of such are 4-methoxysalicylic acid salts. Although the above salts should not specifically be restricted, for example, those of alkaline metal salts or alkaline earth metal salts such as of sodium salts, potassium salts, and calcium salts, and others such as of ammonium salts and amino acid salts. In the present invention, those of potassium salts are preferable and those of 4-methoxysalicylic acid salts are particularly preferable.

The hydroquinone glycosides usable in the present invention include, for example, hexose glycosides such as hydroquinone α-D-glucose, hydroquinone β-D-glucose, hydroquinone α-L-glucose, hydroquinone β-L-glucose, hydroquinone α-D-galactose, hydroquinone β-D-galactose, hydroquinone α-L-galactose, and hydroquinone β-L-galactose; pentaose glycosides such as hydroquinone α-D-ribose, hydroquinone β-D-ribose, hydroquinone α-L-ribose, hydroquinone β-L-ribose, hydroquinone α-D-arabinose, hydroquinone β-D-arabinose, hydroquinone α-L-arabinose, and hydroquinone β-L-arabinose; amino acid glycosides such as hydroquinone α-D-glucosamine, hydroquinone β-D-glucosamine, hydroquinone α-L-glucosamine, hydroquinone β-L-glucosamine, hydroquinone α-D-galactosamine, hydroquinone β-D-galactosamine, hydroquinone α-L-galactosamine, and hydroquinone β-L-galactosamine; and uronic acid glycosides such as hydroquinone α-D-glucuronic acid, hydroquinone β-D-glucuronic acid, hydroquinone α-L-glucuronic acid, hydroquinone β-L-glucuronic acid, hydroquinone α-D-galacturonic acid, hydroquinone β-D-galacturonic acid, hydroquinone α-L-galacturonic acid, and hydroquinone β-L-galacturonic acid.

In view of the level of skin-whitening effect, easiness of availability, and safeness, the hydroquinone glycosides usable in the present invention include, preferably, hydroquinone β-D-glucosides and/or hydroquinone α-D-glucosides, and particularly hydroquinone β-D-glucosides (generally called arbutin and it is called "arbutin" throughout the specification, hereinafter).

Examples of the derivatives of hydroquinone glycosides include esters of acetylated compounds and ethers such as methylated compounds.

The tranexamic acid derivatives usable in the present invention include, for example, a dimmer of tranexamic acid such as hydrochlorate trans-4-(trans-aminomethylcyclohexane-carbonyl)aminomethylcyclohexanecarboxylic acid;

ester of tranexamic acid and hydroquinone, such as trans-4-aminomethylcyclohexanecarboxylic acid 4'-hydroxyphenylester; an ester of tranexamic acid and gentisic acid, such as 2-(trans-4-aminomethylcyclohexylcarbonyloxy)-5-hydroxybenzoic acid and salts thereof; an amide of tranexamic acid such as trans-4-aminomethylcyclohexanecarboxylic acid methylamide and salts thereof; trans-4-(p-methoxybenzoyl)aminomethylcyclohexane-carboxylic acid and salts thereof; and trans-4-guanidino-methylcyclohexanecarboxylic acid and salts thereof.

The resorcinol derivatives usable in the present invention include, for example, alkylresorcinol as a representative example. Concretely, 4-alkylresorcinol is preferable and 4-n-butylresorcinol is particularly preferable.

Any kojic acid and derivatives thereof can be used in the present invention in dependently of their origins and preparation methods, and examples of such derivatives include kojic acid esters such as kojic acid alkyl ester, kojic acid ethers such as kojic acid alkyl ethers, and kojic acid glycosides. Concrete examples of such kojic acid ethers include 2-methoxymethyl-5-hydroxy-4H-pyrane-4-on, 2-ethoymethyl-5-hydroxy-4H-pyrane-4-on, 2-benzoyloxymethyl-5-hydroxy-4H-pyrane-4-on, 2-cinnamoyloxymethyl-5-hydroxy-4H-pyrane-4-on, 2-phenoxymethyl-5-hydroxy-4H-pyrane-4-on, etc. Concrete examples of the above kojic acid esters include kojic acid palmitate, kojic acid stearate, etc. Examples of the above kojic acid glycosides include those which a saccharide residue binds to $-CH_2OH$ at C-2 of kojic acid. Examples of such include hexose such as glucose, galactose, mannose, fructose, and sorbose; pentose such as ribose, arabinose, xylose, lyxose, and xylulose; amino sugars such as glucosamine, mannosamine, and galactosamine; disaccharides such as maltose, lactose, cellobiose, and sucrose; and trisaccharides such as maltotriose and cellotriose. Among which, kojic acid is preferable because of its strong enhancement effect on skin-whitening action, etc.

The chamomile extracts usable in the present invention include those which are prepared by extracting chamomile with an appropriate solvent. Also the chamomile extracts can be used after being processed with concentration, deodorization, purification, drying, etc., as long as they do not spoil the effects and functions of the present invention.

The tetrahydrocurcuminoids usable in the present invention include, for example, tetrahydrocurcumine, tetrahydrodemethoxycurcumine, and tetrahydrobisdemethoxycurcumine, among which, tetrahydrocurcumine is particularly preferable.

The extract powder of indigo plant of the present invention or the mixture of the extract powder and any of the above-identified other ingredients with skin-whitening action can be used intact as external dermal agents, or they can be incorporated with one or more pharmacologically or physiologically acceptable ingredients other than the above ingredients in an amount within the range that does not spoil the effects and functions of the present invention. Examples of these ingredients include, for example, water, alcohols, oil ingredients, surfactants, antiseptics (antibacterials), flavors, humectants, thickeners, water-soluble high molecules, antioxidants, chelating agents, pH-controlling agents, foaming agents, ultraviolet ray-absorbing/scattering agents, powders, vitamins, amino acids, anti-inflammatories, seaweed extracts, additives for pharmaceuticals/quasi-drugs/cosmetics/food products, and effective ingredients for pharmaceuticals/quasi-drugs.

Concrete examples of such are plant oils and fats such as macadamia nut oil, castor oil, olive oil, cacao oil, camellia oil, palm oil, vegetable wax, jojoba oil, grape seed oil, and avocado oil; animal fats and oils such as mink oil and egg yolk oil; waxes such as beeswax, whale wax, lanoline, carnauba wax, and candelilla wax; hydrocarbons such as liquid paraffin, squalene, microcrystalline wax, ceresin wax, paraffin wax, and petrolatum; natural and synthetic fatty acids such as capric acid, myristic acid, palmitic acid, stearic acid, behenic acid, lanolin fatty acid, linoleic acid, linolenic acid, lauric acid, myristic acid, oleic acid, and isostearic acid; natural and synthetic high alcohols such as cetanol, stearyl alcohol, hexyl decanol, octyl dodecanol, lauryl alcohol, capryl alcohol, myristyl alcohol, cetyl alcohol, cholesterol, and phytosterol; and esters such as isopropyl myristate, isopropyl palmitate, octyldodecyl myristate, octyldodecyl oleate, and cholesterol oleate.

Examples of the above surfactants include non-ionic surfactants such as sorbitan monolaurate, sorbitan monopalmitate, sorbitan sesquioleate, sorbitan trioleate, polyoxyethylene sorbitan monolaurate, polyoxyethylene sorbitan monostearate, polyoxyethylene glycol monooleate, polyoxyethylene glycol alkylate, polyoxyethylene alkyl ether, polyglycol diether, lauroyl diethanol amide, fatty acid isopropanol amide, maltitol hydroxy fatty acid ester, alkylating polysaccharide, alkyl glucoside, and sugar ester; nonionic surfactants such as glycerin monostearate lipophilic, glycerin monostearate self-emulsifying, polyglycerin monostearate, polyglycerin alkylate, sorbitan monooleate, polyethyleneglycol monostearate, polyoxyethylene sorbitan monooleate, polyoxyethylene cetylether, polyoxyethylated sterol, polyoxyethylated lanoline, polyoxyethylated beeswax, and polyoxyethylene hydrogenated castor oil; anionic surfactants such as sodium stearate, potassium palmitate, sodium cetyl sulfate, sodium lauryl phosphate, triethanolamine palmitate, sodium polyoxyethylene lauryl phosphate, sodium N-acylglutamate, sodium palmitate, sodium laurate, sodium laureth sulfate, potassium laureth sulfate, triethanolamine ether alkyl sulfate, sulfated castor oil, linear-dodecyl-benzenesulfonate, polyoxyethylene hydrogenated castor oil maleate, and N-acyl-N-methyl taurate; cationic surfactants such as stearyldimethylbenzenzylammonium chloride, stearyltrimethyl-ammonium chloride, stearyl trimethyl ammonium chloride, benzalkonium chloride, and laurylamineoxide; amphoteric surfactants such as alkylaminoethylglycine solution, and lecithin.

Examples of the antiseptics (antibacterials) include benzoic acid and salts thereof, salicylic acid and salts thereof, sorbic acid and salts thereof, dehydroacetic acid and salts thereof, p-oxybenzoates such as p-oxyalkylester benzoate, 2,4,4'-trichloro-2'-hydroxydiphenylether, 3,4,4'-trichlorocarbanilide, hexachlorophene, benzalkonium chloride, phenoxyethanol, hinokitiol, resorcin, ethanol, 1,3-buyleneglycol, and Kankoso 201.

Examples of the flavors include benzaldehyde, benzylbenzoate, phenylacetate, santalol, eugenol, lilial, lyral, linalol, 2-methyl-3-(4-methylphenyl)-propanal, musk ketone, cinnamic aldehyde, beltfix, methylionone, geraniru formate, ISO E SUPER®, γ-undecalactone, hexyl salicylate, cis-3-hexenyl-salicylate, methyl dihydrojasmonate, tetrahydrofurfryl 3-mercaptopropionate, covanol, vanillin, vanillal, geranium oil, pennyroyal oil, birth oil, and armoize oil. Since these flavors mask unfavorable smell inherent to plant extracts including the liquid extract of the present invention, they can be advantageously used to improve the smell of the external dermal agent of the present invention.

Examples of the humectants include polyalcohols such as glycerin, erythritol, xylitol, maltitol, glycerin, propylene glycol, 1,3-butyleneglycol, sorbitol, maltitol, polyglycerin, polyethylene glycol, dipropylene glycol, 1,2-pentanediol, and isoprene glycol; saccharides such as glucose, maltose, trehalose, saccharide derivatives of trehalose, dextrin, cyclodextrin, isocyclodextrin, cyclonigerosylnigerose, cyclomaltosylmaltose, and cyclic saccharides including cyclic penta-/hexa-saccharides such as cyclo$\{\rightarrow 6\}$-[$\alpha$-D-glucopyranosyl-(1-4)]n-$\alpha$-D-glucopyranosyl-(1$\rightarrow$} (n means 4 or 5); NMF (natural moisturizing factors) such as amino acids, peptides, sodium lactate, and sodium pyrrolidone carboxylate; soluble high molecules such as xyloglucan, quince seed, carrageenan, pectin, mannan, curdlan, galactan, dermatan sulfate, glycogen, keratan sulfate, chondroitin, chondroitin sulfate, mucoitin sulfate, keratosulfate, locust bean gum, succinoglucan, caronic acid, hyaluronic acid, heparan sulfate, sodium hyaluronate, collagen, and mucopolysaccharide; silicons such as dimethyl polysiloxane and methylphenylsiloxane; culture supernatants such as lactic acid bacteria and bifidobacteria; and plant extracts such as of Tasmanian blue gum (Eucalyptus *globulus*), hop, ginger, *Uncaria gambier* Roxb., *Rosa multiflora* Thunb., *Aesculus hippocastanum* L., *Coix lachryma-jobi* L., *Typha* latifolia, Japanese medlar, *Panax ginseng*, *Saponaria officinalis, Betula platyphylla* var. japonica, *Hydrangea macrophilla* var. thunbergii, *Syzygium aromaticum* Merr. et Perry, *Carthamus tinctorius, Sanguisorba officinalis*, plants of the genus *Iris, Tilia miqueliana*, plants of the genus *Hamamelis, Paeonia suffruticosa*, and *Thujopsis dolabrata*. Among which, one or more of trehalose, saccharide derivatives of trehalose, cyclic tetrasaccharides, hyaluronic acid, salts of hyaluronic acid, chondroitin sulfate, and salts of chondroitin sulfate, which all have a relatively strong moisturizing action.

Examples of the thickeners include natural high molecular substances such as sodium alginate, xanthan gum, aluminum silicate, extract of seeds of *Pyrus Cydonia*, gum arabic, hydroxyethyl guar gum, carboxymethyl guar gum, guar gum, dextran, tragacanth gum, cellulose, starch, pullulan, chitin, chitosan, carboxymethyl chitin, and agar; semisynthetic high molecular substances such as hydroxypropylcellulose, methylhydroxypropylcellulose, methylcellulose, carboxymethylcellulose, hydroxyethylcellulose, soluble starch, branched starch, and cationized cellulose: synthetic macromolecular substances such as carboxy vinyl polymer, poly(vinyl alcohol), poly(vinylpyrroridone), and vinyl alcohol/vinyl acetate copolymers.

Examples of the antioxidants include dibutylated hydroxytoluene, dibutylhydroxyanisol, gallic acid propyl, L-ascorbic acid, vitamin E, vitamin P, catechins, flavonoids, and derivatives thereof.

Examples of the chelates include disodium edetate, ethylenediaminetetraacetate, pyrophosphate, salts of hexametaphosphoric acid, citric acid, tartaric acid, and gluconic acid.

Examples of the pH-controllers include sodium hydroxide, potassium hydroxide, triethanolamine, nitrotriethanol, citric acid, sodium citrate, boric acid, borax, and potassium hydrogen phosphate.

Examples of the ultraviolet ray absorbing/scattering agents include p-aminobenzoic acid ultraviolet ray absorbers, anthranilic acid ultraviolet ray absorbers, salicylic acid ultraviolet ray absorbers, cinnamic acid ultraviolet ray absorbers, benzophenone ultraviolet ray absorbers, saccharide ultraviolet ray absorbers, 3-(4'-methylbenzylidene)-*d*-camphor, 3-benzylidene-*d,l*-camphor, urocanic acid, ethyl ester of urocanic acid, 2-phenyl-5-methylbenzoxazole, 2,2'-hydroxy-5-methyl phenylbenzotriazole, 2-(2'-hydroxy-5'-*t*-octylphenyl)benzotriazole, 2-(2'-hydroxy-5'-hydroxy-5'-methylphenyl)benzotriazole, dibenzalazin, dianisoyl methane, 4-methoxy-4'-*t*-butylbenzoyl-methane, 5-(3,3-dimethyl-2-norbor-nyliden)-3-pentene-2-on, 2-hydroxy-4-methoxybenzophenone, octyldimethylamino-*p*-benzoate, ethylhexyl-*p*-methoxycinnamate, titanium oxide, kaolin, and talc.

Examples of the vitamins, excluding L-ascorbic acid and derivatives thereof, include vitamin A; derivatives of vitamin A; vitamins belonging to vitamin B such as vitamin $B_1$, derivatives of vitamin $B_1$, vitamin $B_2$, derivatives of vitamin $B_2$, vitamin $B_6$ hydrochlorides, vitamin $B_6$ tripalmitate, vitamin $B_6$ dioctanoate, vitamin $B_{12}$, vitamin $B_{15}$, and derivatives thereof; vitamins belonging to vitamin D; vitamins belonging to vitamin E including $\alpha$-tocopherol, $\beta$-tocopherol, $\gamma$-toco-pherol, and vitamin E acetate; vitamin H; pantothenic acid; pantethine; vitamin F; vitamin K; bioflavonoids such as rutin/hesperidin/naringin and derivatives thereof; vitamin U, ferulic acid, $\gamma$-orizanol; $\alpha$-lipoic acid; orotic acid; coenzyme $Q_{10}$; and derivatives thereof.

Examples of the amino acids include glycine, alanine, valine, leucine, isoleucine, serine, threonine, phenylalanine, tyrosine, asparagine, glutamine, taurine, tryptophane, cystine, cysteine, methionine, proline, hydroxyproline, aspartic acid, glutamic acid, arginine, histidine, lysine, carnitine, and derivatives thereof, as well as salts thereof.

Examples of the anti-inflammatories include allantoin and derivatives thereof such as allantoin, allantoin N-acetyl-*dl*-methionine, chlorohydroxyaluminum allantoinate, dihydroxyaluminum allantoinate, and polygalacturonic acid allantoinate; glycyrrhetin and derivatives thereof such as glycyrrhetinic acid, glycyrrhizin, allantoin glycyrrhetinic acid, glyceryl glycyrrhetinate, stearyl glycyrrhetinate, glycyrrhetinyl stearate, disodium 3-succinoyl glycyrrhetinate, dipotassium glycyrrhizinate, and monoammonium glycyrrhizinate; pantothenic acid and derivatives thereof such as pantothenyl alcohol, pantothenylethylether, acetylpantothenylethylether, panthenylethylether acetate, calcium pantothenate, sodium pantothenate, acetylpantothenylethylether, pantothenylethylether benzoate, and pantethine; vitamin E and derivatives thereof such as d-δ-tocopherol, *dl*-α-tocopherol, *dl*-α-tocopherol acetate, *d*-δ-tocopherol linoleate, *dl*-α-tocopherol nicotinate, and *dl*-α-tocopherol succinate; L-ascorbic acid and derivatives thereof including L-ascorbic acid glycosides such as L-ascorbic acid 2-glucoside, acylated derivatives of such glycosides, ascorbyl tetrahexyldecanoate, asborbyl tocopherol phosphodiester where L-ascorbic acid and tocopherol are linked together via phosphoric acid residue, L-ascorbic acid sulfate, ascorbyl dipalmitate, L-ascorbyl palmitate, L-ascorbyl stearate, L-ascorbyl phosphate, ethyl ascorbic acid, and acylated derivatives thereof, as well as alkali and alkali earth metal salts thereof; pyridoxine hydrochloride, menthol, biotin, camphor, turpentine oil, zinc oxide, azulene, guaiazulene, and derivatives thereof; mefenamic acid and derivatives thereof; phenylbutazone and derivatives thereof; indomethacin and derivatives thereof; ibuprofen and derivatives thereof; ketoprofen and derivatives thereof; ε-aminocapronic acid, diclofenac sodium, diphenhydramine, tranexamic acid, and derivatives thereof; adrenal cortex hormones such as dexamethasone, cortisone, esters of cortisone, hydrocortisone, esters of hydrocortisone, prednisone, and prednisolone; antihistamine agents; and plants and ingredients derived therefrom such as *Rosae Multiflorae Fructus, Polygonum bistorta, Curcumae Rhizoma, Hypericaceae*, cork tree bark, sweetroot, *Lonicera japonica, Nasturtium officinale, Russian comfrey, Acanthopanax gracilistylus, Salvia splendens, Lithospermi Radix, Betula platyphylla Sukatschev* var. japonica, tea tree, *Calendula arvensis* L., *Sambucus racemosa* L. subsp. sieboldiana, plants of the family *Typhaceae, Sapindus mukurossi* Gaertn., extracts of eucalypti, *Brassica oleracea* L. var. italica Plenck (broccoli), *Angelica acutiloba*, leaves of Japanese medlar, Japanese basil, *Matricaria chamomilla* L., *artemisia, Aloe barbadensis* miller, *Daucus carota* L., powdered *Phellodendri Cortex*, powdered of Myrica *rubra* Sieb. et Zucc, cube gambir, Hydrangea *macrophilla* var. thunbergii, *Althaea*, arnica, *Echinacea purpurea, Plectranthus japonicus, Scutellariae Radix, Hordeum vulgare,* Saint John's wort, orange, valerian, *Anthemis nobilis, Artemisia capillaris* Thunb, *Cucumis sativus*, cape jasmine, Sasa *veitchii* (Carr.) Rehd, gentian, *Geranium thunbergii, Arctium lappa* L., *Russian comfrey*, Japanese pepper, *Perilla frutescens, Tilia miqueliana* Maxim, Chinese peony, *Hedera helix* L., *Achillea millefolium, Mentha piperita, Cnidii Rhizoma, Swertia japonica, Salvia officinalis*, "*Sohakuhi*" (a plant of the family *Mulberry*), *Zizyphi Fructus,* thyme, *Benincasae Semen, Calendula officinalis, Persicae semen, Houttuynia cordata* Thunb, *Potentilla tormentila* Neck, carrot (*Daucus carota* L.), *Petroselium crispum*, mint, *Urtica thunbergiana, Santalum album* L., Japanese medlar, *Ruscus aculeatus* L., grapes, *Carthamus tinctorius*, Japanese tree peony, *Ficus religiosa*, horse chestnut, *Amygdalus persica, Rodgersia podophylla, Artemisia princeps* Pamp., lavender, *Rosmarinus officinalis* L., Japanese medlar, carrot, and *Angelica acutilaba.*

Examples of the seaweed extracts include extracts of brown alga, red alga, green alga, and blue-green alga; and concrete examples of such include those of tangles, *Laminaria japonica, Undaria pinnatifida, Hizikia fusiformis Okam, Gelidium amansii, Corallina Officinalis, Corallina officinlis* Linné, alge of the genus Palmaria, Chondrus ocellatus Holmes, marine alge, sea lettuce, *Ulva pertusa* Kjellman, *Ascophyllum nodosum, Fucus evanescens* C. Agardh, *Nemacystus decipiens, Cladosiphon okamuranus*, and *Himanthalia*. Extracts of aquatic plants such as *Zostera marina* L. are included in the above.

In addition to the above-identified ingredients, the following can be incorporated into the external dermal agents incorporated with the extract powder of indigo plant of the present invention: One or more of appropriate ingredients such as substances which have blood circulation promoting action, astringent action, anti-wrinkle action, cell-activating action, anti-ageing activity, hair-growth-promoting activity, hair-regeneration-promoting activity, and percutaneous-absorption-accelerating action. Examples of such include plant and animal ingredients other than indigo plant, such as propolis, herbs including Chinese parsleying, and extracts derived therefrom; inorganic powders of deep seawater, bittern, minerals such as bittern ingredients, antibiotics, calcium dihydrogen phosphate, organo-metamorphic montmorillonite, silicic acid, anhydrous silicic acid, magnesium silicate, mica, bentonite, titanium-coated mica, bismuth oxychloride, zirconium oxide, magnesium oxide, zinc oxide, calcium carbonate, magnesium carbonate, ferric oxide, ultramarine blue, iron blue pigment, chrome oxide, chrome hydroxide, calamine, zeolite, and carbon black; organic powders such as polyamide, polyester, polyethylene, polypropylene, polystyrene, polyurethane, vinyl resin, urea resin, phenol resin, fluororesin, silicon resin, acrylic acid resin, melamine resin, epoxy resin, polycarbonate resin, divinylbenzene-styrene copolymer, copolymers of two or more monomers of any of the above compounds, proteins including silk, scleroprotein, and cellulose; natural colors such as anthraquinone, anthocyanin, chalkone, and carotenoid pigments/dyes; photosensitizing dyes such as Kankoso 101, Kankoso 201, Kankoso 301, and Kankoso 401; organic color powders such as Red No. 201, Red No. 202, Red No. 204, Red No. 205, Red No. 220, Red No. 226, Red No. 228, Red No. 405, Orange No. 203, Orange No. 204, Yellow No. 204, Yellow No. 401, and Blue No. 404; organic color powders of zirconium, barium or aluminum lake such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and powders of the above-identified organic color powders to which are supported any one of colors such as natural and organic synthetic dyes containing purple-blue dye or safflower dye, or of functional ingredients such as glycosyl rutin, glycosyl hesperidin, glycosyl glycyrrhizin, glycosyl naringin, esculetin, esculin, and glycosyl esculin.

The amount of the above-identified substances to be incorporated into the external dermal agents should not specifically be restricted as long as it does not spoil the properties of the objective external dermal agents and/or effects and functions of the present invention, and it can be arbitrarily adjusted depending on their use and usually preferably adjusted to 0.0001 to 99% to the total amount of any one of the external dermal agents, desirably, 0.001 to 70%, more desirably, 0.01 to 30%.

The forms of the external dermal agents incorporated with the extract powder of indigo plant of the present invention are arbitrary, for example, any forms of solubilized systems such as powders, solids, and cosmetic lotions; emulsified systems such as milky lotions, creams, and pastes; or other systems of ointments and dispersion solutions can be selected.

The term external dermal agents as referred to as in the present invention means external preparations applied externally or intraorally to the skin or the scalp; ointments, creams, milky lotions, lotions, extracts, jellies, gels, packs, masks, bath salts, powdered dentifrices, moistened dentifrices, toothpastes, liquid dentifrices, medical dentifrices, cachous, cachou films, mouthwashes, and gargles, including those which are used for animals and pets.

The extract powder of indigo plant of the present invention can be orally ingestible intact or it can be arbitrarily used in preparing orally ingestible compositions such as food products, quasi-drugs, pharmaceuticals, feeds, baits, pet foods after mixed with one or more of reducing saccharides, non-reducing saccharides, sugar alcohols, sweeteners with high sweetening power, water-soluble polysaccharides, organic acids, inorganic acids, salts, emulsifiers, antioxidants, substances with chelating action, conventional colors, flavors, preservatives, acids, taste-imparting agents, sweeteners, stabilizers, fillers, alcohols, and water-soluble high molecules. The orally ingestible compositions containing the extract powder of indigo plant exert the physiological activities inherent to the extract powder, when perorally ingested. In this case, similarly as dentifrices, cachous, and mouthwashes, the above-identified orally ingestible compositions exert the prevention or the improvement of periodontal diseases by keeping the strength, elasticity, and color of gums intraorally. Explaining the case of incorporating the extract powder of indigo plant into orally ingestible compositions including food products, pharmaceuticals, and quasi-drugs, wherein the food products include seasonings and sweeteners, for example, amino acids, peptides, soy sauces, powdered soy sauces, "*miso*" (a soybean paste), powdered "*miso*", unrefined "sake"/soy sauces, salted meat, "*furikake*" (a ready-made topping for seasoning cooked rice), mayonnaises, dressings, powdered sweetened vinegars, premixes for Chinese foods, sauces, ketchups, sauces for grilled meats, curry blocks, premixes of stews, premixes of soups, "*dashi-no-mono*" (a soup concentrate), nucleoside seasonings, mixed seasonings, "*mirin*" (a sweet sake), "*shin-mirin*" (a sweet sake), table sugars, and coffee sugars.

Further, the above-identified food products include, for example, rice confectioneries such as rice crackers, cubic rice crackers, and "*okoshi*" (a millet-and-rice cake); Japanese confectioneries such as "*gyuhi*" (a starch paste), "*monaka*" (a bean-jam-filled-wafer), rice cakes, "*ohagi*" (a rice dumpling covered with bean jam), "*manju*" (a steamed bean-jam bun), "*karukan*" (a sweetened jelly of yam and rice flour), "uiro" (a steamed rice with sugar), "an" (a bean's paste) and related products, "yokan" (a sweet jelly of beans), "*mizu-yokan*" (a soft adzuki-bean jelly), "*kingyoku*" (a Japanese unbaked cake), "*kintsuba*" (a Japanese baked cake with bean's paste and wheat flour), sweet potato, jellies, bavarois, castellas, and candies; baked confectioneries such as biscuits, cookies, crackers, pies, cream puffs, wafers, sponge cakes, doughnuts, and pastries; Western confectioneries including soft candies, hard candies, fondants, and icings, such as puddings, butter creams, custard creams, chocolates, chewing gums, nougats, jelly beans, gummy candies, caramels, and marshmallows; snacks; cereals; center liquid confectioneries; meringue confectioneries; breads such as sandwich loafs, bread rolls, buns filled with bean pastes, and muffins; fruit pickles; syrups such as "*korimitsu*" (a concentrated saccharide syrup); pastes such as flour pastes, peanut pastes, fruit pastes, and spreads; processed fruits such as jams, marmalades, preserves, foods preserved in syrups, candied fruits, and cut fruits; budbreak vegetables such as bean sprouts, alfalfas, and broccoli sprouts; vegetable juices such as green juices; processed foods of vegetables such as cut vegetables, salads, and cooked vegetables; pickled vegetable and premixes used therefor such as premixes for fresh vegetables for preparing pickled vegetable; rice products such as plain cooked rice, rice balls, rice with red beans, rice gruels, seasoned rice for *sushi*, seasoned cooked rice, and gelatinized rice; processed bean products such as soybean milk, bean curd, freeze-dried bean curd, and "*nattou*" (a fermented soybean); noodles such as Japanese wheat noodles, Japanese buckwheat noodles, Chinese noodles, and pastas; "*okonomi-yaki*" (a Japanese-style pancake containing vegetables and other foodstuff), "*tako-yaki*" (a wheat flour ball with cut octopus), "*tai-yaki*" (a fish-shaped cake filled with red bean paste), croquette, dumpling, *shao-mai*, spring rolls; processed meat and fish meat products such as hams and sausages, fish hams, fish sausages, "*kamaboko*" (a fish minced and steamed), "*chikuwa*" (a fish paste), and "*tempura*" (a Japanese deep-fried food); food delicacies such as salted sea urchin and squid, "*su-konbu*" (a seasoned tangle with vinegar), "*saki-surume*" (a torn dried squid), "*fugu-no-mirin-boshi*" (a dried swellfish seasoned with *mirin*), salmon caviar, and toasted and seasoned laver; bastes and sauces used for seasoning grilled meat, grilled eel, rice dumpling, and Japanese cracker; foods boiled down in soy sauces produced with lavers, wild vegetables, dried cuttlefish, little fish, and shellfish; daily dishes such as boiled beans, potato salads, and tangle rolls; eggs and processed egg products such as fried eggs, and steamed egg hotchpotches; dairy products such as cheeses and yogurts; fish meats, animal meats, fruits, vegetables, and their freezed products, chilled products, pouch-packed foods, dried foods, and freeze-dried foods; bottled vegetables; canned foods; food mixes such as pudding mixes, hot cake mixes, and butter mixes; alcohols such as sakes, synthetic sakes, liqueurs, whiskeys/wines, beers, and sparkling liquors; soft drinks such as green teas, teas, coffees, cocoas, juices, carbonated beverages, lactic beverages, and lactobacillus beverages. The pharmaceuticals and quasi-drugs usable in the present invention include those in the form of a solid (a tablet, powdered medicine, or granule), paste, syrup, or liquid; and feeds, baits, pet foods for animals and pets.

With reference to the following Examples of Experiments, the extract powder of indigo plant of the present invention and the effects and functions thereof are explained in more detail:

<Experiment 1: Relationship between the stability of extract powder of indigo plant and the DE of partial starch hydrolyzate used as a base for pulverization>

There exists a problem that, when a liquid extract of indigo plant is dried alone or after admixed with sugar alcohol(s), the resulting dried product absorbs moisture and solidifies just after its processing even when placed under a relative humidity of 53% at 25°C. Using a partial starch hydrolyzate as a base for pulverization to pulverize a liquid extract of indigo plant, it was examined the influence of the partial starch hydrolyzate on the stability of the resulting extract powder of indigo plant. The following experiment was conducted to examine the influence of the DE of the partial starch hydrolyzate used as a base for pulverization.

<Preparation of liquid extract of indigo plant>

In accordance with the method of the later described Example 1, one part by weight of a dried leaf of *Polygonum tinctorium* Lour. was pulverized, placed in a jacketed-tank, admixed with 24 parts by weight of water, and stirred while heating to obtain a liquid extract of indigo plant extracted with hot water. The liquid extract was filtered by "Ultrafiltration Module MICROZA API2013", a UF membrane commercialized by Asashikasei Co., Ltd., Tokyo, Japan, to obtain a liquid extract of indigo plant with a solid content of one percent.

<Preparation of partial starch hydrolyzate solutions with different DEs used as bases for pulverization>

To a 2% starch suspension was added 0.5, 1, 3, 5, 8, 10, 15, 30 or 40 units/g starch of "TERMAMYL", a product name of $\alpha$-amylase produced by Novo Industri A/S, Copenhagen, Denmark, kept at 90 to 95° C for gelatinization and liquefaction, heated to inactivate the remaining $\alpha$-amylase, and then filtered, purified and concentrated in usual manner to obtain a solution containing partial starch hydrolyzate with a DE of 0.1, 0.2, 1.5, 2.5, 4.1, 5.2, 8.0, 10.2 or 13.5.

<Preparation of extract powder of indigo plant>

The liquid extracts of indigo plant prepared by the above method were concentrated to give an about 10-time strength, and the concentrates were measured for their solid concentrations and admixed with any of the above solutions containing partial starch hydrolyzates with different DEs in a weight ratio of 1:2 (the solid content, d.s.b., of any one of the liquid extracts):(the solid content, d.s.b., any one of the solutions containing partial starch hydrolyzates), and spray-dried in usual manner to obtain an extract powder of indigo plant with a moisture content of less than 4%.

<Evaluation method>

The above respective powders were placed in aluminum vessels, stored under a relative humidity of 53% at 25° C for five days, evaluated for their floatability and solidification, and judged for easiness of handleability as powders. The results are in Table 1. The floatability was judged based on the following four ranks: "+++", being dried and high in floatability; "++", having satisfactory floatability; "+", being low in floatability but still retaining it; and "-", being moisturous and free of fluidability. The solidification was judged based on the two ranks in terms of the presence "+" or the absence "-" of lump or clump. When the liquid extract of indigo plant was spray-dried alone without addition of any one of the partial starch hydrolyzates, the resulting extract powders were strongly high in hygroscopicity and could not be handled as powders.

[Table 1]

| Starch (DE) | Fluidability | Solidification | Judgement |
|---|---|---|---|
| 0.1 | Too high in liquid viscosity and hard to spray | | |
| 0.2 | +++ | + | Excellent |
| 1.5 | +++ | + | Excellent |
| 2.5 | +++ | + | Excellent |
| 4.1 | +++ | + | Excellent |
| 5.2 | ++ | + | Excellent |
| 8.0 | + | + | Good |
| 10.2 | + | + | Passable |
| 13.5 | - | - | Impassable |

As evident from Table 1, when stored under a relative humidity of 53% at 25° C for five days, the extract powders of indigo plant prepared by pulverizing the liquid extract of indigo plant using the partial starch hydrolyzates with a DE of 10.2 or lower gave no hygroscopicity, had a satisfactory fluidability, and gave no formation of lump or clump, and thus their handling as powders were judged passable. When pulverized after addition of partial starch hydrolyzates with a DE of 8.0 or lower, the obtained extract powders gave an increased fluidability and an improved handleability and they gave the best properties when pulverized with partial starch hydrolyzates with a DE of 5.2 or lower. While, when partial starch hydrolyzates with a DE of 0.1 were added to the liquid extracts, the viscosity of the resulting solutions became so high as to spoil spray-drying, resulting in a judgement that the DE suitable for spray-drying of amylaceous saccharides treated with amylase is 0.2 or higher.

<Experiment 2: Relationship between the stability of extract powder of indigo plant and the ratio of partial starch hydrolyzate, which may influence on the stability, used as a base for pulverization>

It was conducted the following experiment to examine the relationship between the stability of extract powder of indigo plant and the ratio of partial starch hydrolyzate, which may influence on the stability, used as a base for pulverization.

<Preparation of liquid extract of indigo plant>

The liquid extract of indigo plant prepared in Experiment 1 was used.

<Preparation of solutions containing partial starch hydrolyzates with different DEs used as bases for pulverization>

The solution containing a partial starch hydrolyzate with a DE of 1.5, 4.1, or 5.2, prepared in Experiment 1, was used as a base for pulverization. In addition to these, "DEXPEARL SD-20" with a DE of 1.4, a product name of a commercialized partial starch hydrolyzate containing cyclodextrin commercialized by Ensuiko Sugar Refining Co., Ltd., Tokyo, Japan, was used as a base for pulverization.

<Preparation of extract powder of indigo plant>

The above indigo plant extract was concentrated to give a 10-time strength and admixed with any of the solutions containing partial starch hydrolyzates in an amount that the weight ratio of the partial starch hydrolyzate, d.s.b., of any of the solutions to one part by weight of the solid content of the above concentrate was made as shown in Table 2, and spray-dried in usual manner to obtain extract powders of indigo plant with moisture contents of less than four percent.

<Evaluation method>

Similarly as in Experiment 1, the respective powders obtained in the above were placed in aluminum vessels, stored under a relative humidity of 53% at 25° C for five days, and then evaluated for fluidability and solidification and judged for easiness of handleability as powders. The results are in Table 2. The evaluations of fluidability and solidification were conducted based on the same criterion as in Experiment 1.

[Table 2]

| Amylaceous substance : (DE) | (Solid content of indigo plant extract) (Partial starch hydrolyzate) (weight ratio, d.s.b.) | Fluidability | Solidification | Judgement |
|---|---|---|---|---|
| With no addition | - | - | - | Impassable |
| 1.5 | 1:0.1 | - | - | Impassable |
| | 1:0.25 | + | + | Passable |
| | 1:0.5 | ++ | + | Good |
| | 1:1 | +++ | + | Excellent |
| | 1:2 | +++ | + | Excellent |
| | 1:5 | +++ | + | Excellent |
| | 1:7.5 | +++ | + | Excellent |
| | 1:10 | +++ | + | Excellent |
| 3.5 | 1:0.1 | - | - | Impassable |
| | 1:0.25 | + | + | Passable |
| | 1:0.5 | ++ | + | Good |
| | 1:1 | +++ | + | Excellent |
| | 1:2 | +++ | + | Excellent |
| | 1:5 | +++ | + | Excellent |
| | 1:7.5 | +++ | + | Excellent |
| | 1:10 | +++ | + | Excellent |
| 5.2 | 1:0.1 | - | - | Impassable |
| | 1:0.25 | + | + | Passable |
| | 1:0.5 | ++ | + | Good |
| | 1:1 | +++ | + | Excellent |
| | 1:2 | +++ | + | Excellent |
| | 1:5 | +++ | + | Excellent |
| | 1:7.5 | +++ | + | Excellent |
| | 1:10 | +++ | + | Excellent |
| Saccharide containing $\alpha$-cyclodextrin (DE 1.4) | 1:0.1 | - | - | Impassable |
| | 1:0.25 | + | + | Passable |
| | 1:0.5 | + | + | Passable |
| | 1:1 | +++ | + | Excellent |
| | 1:2 | +++ | + | Excellent |
| | 1:5 | +++ | + | Excellent |
| | 1:7.5 | +++ | + | Excellent |
| | 1:10 | +++ | + | Excellent |

As evident from Table 2, in the case of using any of the partial starch hydrolyzates, the extract powders of indigo plant, prepared by pulverizing the indigo plant extract after adding at least 0.25 part by weight of any of the partial starch hydrolyzates to one part by weight of the indigo plant extract, had a satisfactory fluidability and had no formation of any lump/clump or solidification, and thus they were judged to be handleable as powders. These powders became more easily handleable when pulverized after adding at least 0.5 part by weight of any of the partial starch hydrolyzates to one part by weight of the indigo plant extract, and they became most easily handleable when pulverized after adding at least one part (equivalent part) by weight of any of the partial starch hydrolyzates. When pulverized after adding, on a dry solid basis, at least 7.5 parts by weight of any of the partial starch hydrolyzates to one part by weight of the indigo plant extract, the percentage of the partial starch hydrolyzates in the resulting powders became so high as to lower the content of the indigo plant extract in the powders and also increases the frequency of affecting the properties of compositions. Thus, it was judged that, a preferable ratio, d.s.b., of a partial starch hydrolyzate to one part by weight of the indigo plant extract is not higher than five parts by weight, particularly, not higher than 2.5 parts by weight.

<Experiment 3: Influence of extract powders of plants of the family *Polygonaceae* on the melanin formation (test 1 for *in vitro* whitening effect)>

The following experiment was conducted to examine the influence of extract powders of plants of the family *Polygonaceae* on the melanin formation. An extract powder of *Polygonum tinctorium* Lour., Japanese knotweed, buckwheat, or knotgrass as a plant of the family *Polygonaceae* was prepared for examining the influence on the melanin formation.

<Preparation of extract powder of a plant of the family *Polygonaceae*>

Using a dried leaf of *Polygonum tinctorium* Lour., Japanese knotweed, buckwheat, or knotgrass as a plant of the family *Polygonaceae*, extract powders of which were prepared. The extract powders of indigo plant were prepared by the method in later described Example 2. According to the same method as in Example 2, using a dried leaf of Japanese knotweed, buckwheat, or knotgrass in place of the one of *Polygonum tinctorium* Lour., extract powders of the above plants were prepared for use in the following experiment, where, as an extract powder of indigo plant, the one prepared by the method in later described Example 2 was used, unless specified otherwise.

<Experimental method>

$2.2 \times 10^5$ cells of mouse melanoma B16 cells were cultured for 24 hours in usual manner, and the resulting culture was admixed with any of the extract powders of indigo plant to give a concentration of 0.025% or 0.05% and further cultured for 72 hours. After counting the number of viable cells, the viable cells were packed into a capillary tube for color observation. Using a culture medium supplemented with the extract powder of Japanese knotweed, buckwheat, or knotgrass to give a concentration of 0.05%, or supplemented with kojic acid as a positive control to give a concentration of 2.5 mM, mouse melanoma B16 cells were cultured and observed for their color, similarly as the method used in the extract powder of indigo plant. The degree of blackening of the B16 cells was evaluated based on the following criterion, and the results are in Table 3.

(Criterion)

Evaluations with reference to the blackening degree of control free of agent:

- : Unchanged;
± : Slight inhibition of blackening;
+ : Positive inhibition of blackening;
++ : Clear inhibition of blackening;
+++ : Complete inhibition of blackening.

[Table 3]

| Medium composition | Judgement |
| --- | --- |
| Medium supplemented with saccharide containing 0.05% cyclodextrin | - |
| Medium containing 0.025% extract powder of indigo plant | + |
| Medium containing 0.05% extract powder of indigo plant | ++ |
| Medium containing 0.05% extract powder of Japanese knotweed | ± |
| Medium containing 0.05% extract powder of buckwheat | ± |
| Medium containing 0.05% extract powder of knotgrass | ± |
| Medium containing 2.5 mM kojic acid | + |

As evident from Table 3, the cells, cultured in the medium supplemented with 0.025% or 0.05% of the extract powder of indigo plant used in this experiment, were inhibited the blackening at substantially the same level or higher compared with that of the cells cultured in 2.5 mM kojic acid as a positive control, reveling that the extract powder of indigo plant exerts a strong melanin formation inhibitory action. While in the case of the cells with the extract powder of Japanese knotweed, buckwheat, or knotgrass at the same concentration as in the extract powder of indigo plant extract, there was only observed a slight inhibition of blackening, revealing that the extract powder of indigo plant has a relatively strong inhibitory action on blackening among the results with the same plants of the family *Polygonaceae* as the indigo plant. From the data on the media with extract powders of indigo plant at the concentrations tested, the indigo plant gave no cytotoxicity against mouse melanoma B16 cells and this concluded that the inhibitory action on the blackening of mouse melanoma B16 cells by the extract powder of indigo plant in this experimental system was due to the melanin formation inhibitory action by the extract powder and not by the cytotoxic action thereof.

<Experiment 4: Influence of extract powder of indigo plant and other ingredients with skin-whitening action on melanin formation (test 2 for *in vitro* skin-whitening effect)>

The following experiment was conducted to examine the influence of an extract powder of indigo plant and other ingredients with skin-whitening action on melanin formation. Mouse melanoma B16 cells were cultured for 24 hours, and to the resulting culture was added any one of the test solutions to give a composition ratio (%) of each of the ingredients with skin-whitening effect in Table 4 alone or in combination with 0.025% of an extract powder of indigo plant simultaneously, followed by culturing for 72 hours. The viable cells were counted and packed into a capillary tube for color observation. The results are in Table 4. The judgement of the effects was evaluated based on the same criterion as in Experiment 1. In this experiment system, the effect in the case of culturing the cells with addition of 0.025% of the extract powder of indigo extract was judged to be "+".

[Table 4]

| Ingredients with skin-whitening action | Composition ratio (%) | Judgement | |
|---|---|---|---|
| | | With no extract powder of indigo plant | 1.25% extract powder of indigo plant |
| Sodium L-ascorbate | 2.0 | + | +++ |
| 4-Methoxysalicylic acid potassium salt | 1.0 | + | +++ |
| Kojic acid | 1.0 | + | +++ |
| Arbutin | 0.5 | + | +++ |
| Tranexamic acid | 1.0 | ± | ++ |
| Ellagic acid | 0.5 | ± | ++ |
| 4-n-Butylresorcinol | 0.5 | + | +++ |
| Linoleic acid | 0.5 | ± | ++ |
| Chamomile extract* | 1.0 | + | +++ |
| Tetrahydrocurcumin | 0.5 | ± | ++ |

\* : Extract prepared with ethanol/water (8/2, by weight ratio).
(The composition ratios are expressed on a dry solid basis.)

As evident from the test results on the extract powder of indigo plant in Table 3 and the results in Table 4, all the test specimens, where the extract powder of indigo plant was used in combination with sodium L-ascorbate, 4-methoxysalicylic acid potassium salt, kojic acid, arbutin, tranexamic acid, ellagic acid, 4-n-butylresorcinol, linoleic acid, chamomile extract, or tetrahydrocurcumin, exerted a stronger blackening inhibitory effect than that expected when the extract powder of indigo plant and any of the other skin-whitening ingredients are added in combination, and thus it was judged that the combination of the extract powder of indigo plant and any of the other skin-whitening ingredients synergistically augments the blackening inhibitory effect.

<Experiment 5: Influence of extract powder of indigo plant and other ingredients with skin-whitening action on melanin formation (*in vivo* skin-whitening effect)>

The following experiment was conducted to examine the influence of an extract powder of indigo plant and other ingredients with skin-whitening action on melanin formation. Eleven groups, which respectively consisted of 20 volunteers with dark skin, spot, and freckle, were applied their faces every in the morning and evening with any of the following test cosmetic solutions for three months and thereafter the skin-whitening effect by the solutions were evaluated.

(Prescription of test cosmetic solutions)

A solution, which had been prepared by mixing and dissolving any one of the following ingredients (3), (4) and (9) to (11), and another solution, which had been prepared by mixing and dissolving any one of the following ingredients (1), (2), (5), (6), (7), (8) and (11) were mixed to homogeneity into test cosmetic solutions. As the ingredient (6) of "another ingredients with skin-whitening action", any one of those in Table 5 was added in a composition ratio as described in Table 5. Using these test cosmetic solutions, the skin-whitening effects thereof were evaluated. The results are in Table 5 in parallel.

<Prescription>

|       |      |                                                         | (%)                          |
|-------|------|---------------------------------------------------------|------------------------------|
|       | (1)  | Glycerin                                                | 5                            |
|       | (2)  | 1,3-Butyleneglycol                                      | 6.5                          |
|       | (3)  | Polyoxyethylene sorbitan monolaurate (20 E.O.)          | 1.2                          |
|       | (4)  | Ethyl alcohol                                           | 8                            |
|       | (5)  | Extract powder of indigo plant                          | 0.02                         |
|       | (6)  | Other ingredients with skin-whitening action (Table 5)  | Amounts described in Table 5 |
|       | (7)  | Lactic acid                                             | 0.05                         |
|       | (8)  | Sodium lactate                                          | 0.1                          |
|       | (9)  | 2-Ethylhexyl 4-methoxycinnamate                         | 0.1                          |
|       | (10) | Antiseptic                                              | q.s.                         |
|       | (11) | Flavor                                                  | q.s.                         |
|       | (12) | Refined water                                           | Remaining quantity           |

For comparison, a reference cosmetic solution was prepared according to the same composition prescription, composition ratio, and preparation method as in Experiment 5, except for omitting the extract powder of indigo plant (5) among the prescription of the test cosmetic solution in Experiment 5, and adding any one of the ingredients with skin-whitening action in Table 5 as the agent of (6); and evaluated for skin-whitening effect by the same method as applied to the test cosmetic solutions in Experiment 5. The results are in Table 5 in parallel.

[Table 5]

|                          | Other ingredients with skin-whitening action | Composition ratio of other ingredients with skin-whitening action | Composition ratio of indigo plant extract | Judgement |
|--------------------------|-----------------------------------------------|-------------------------------------------------------------------|--------------------------------------------|-----------|
| Test cosmetic solution 1 | L-Ascorbic acid 2-glucoside                   | 0.5                                                               | 0.5                                        | A         |
| Test cosmetic solution 2 | Potassium 4-methoxysalicylate                 | 0.5                                                               | 0.5                                        | A         |
| Test cosmetic solution 3 | Kojic acid                                    | 0.5                                                               | 0.5                                        | A         |
| Test cosmetic solution 4 | Arbutin                                       | 0.5                                                               | 0.5                                        | A         |
| Test cosmetic solution 5 | Tranexamic acid                               | 0.5                                                               | 0.5                                        | B         |

(continued)

| | Other ingredients with skin-whitening action | Composition ratio of other ingredients with skin-whitening action | Composition ratio of indigo plant extract | Judgement |
|---|---|---|---|---|
| Test cosmetic solution 6 | Ellagic acid | 0.5 | 0.5 | B |
| Test cosmetic solution 7 | 4-n-Butylresorcinol | 0.5 | 0.5 | A |
| Test cosmetic solution 8 | Linoleic acid | 0.5 | 0.5 | B |
| Test cosmetic solution 9 | Chamomile extract* | 0.5 | 0.5 | B |
| Test cosmetic solution 10 | Tetrahydrocurcumin | 0.5 | 0.5 | B |
| Comparison cosmetic solution 1 | Indigo plant extract | 0 | 1 | B |
| Comparison cosmetic solution 2 | L-Ascorbic acid 2-glucoside | 1 | 0 | B |
| Comparison cosmetic solution 3 | Potassium 4-methoxysalicylate | 1 | 0 | C |
| Comparison cosmetic solution 4 | Kojic acid | 1 | 0 | C |
| Comparison cosmetic solution 5 | Arbutin | 1 | 0 | C |
| Comparison cosmetic solution 6 | Tranexamic acid | 1 | 0 | C |
| Comparison cosmetic solution 8 | 4-n-Butylresorcinol | 1 | 0 | C |
| Comparison cosmetic solution 9 | Linoleic acid | 1 | 0 | C |
| Comparison cosmetic solution 10 | Chamomile extract* | 1 | 0 | C |
| Comparison cosmetic solution 11 | Tetrahydrocurcumin | 1 | 0 | C |

* : Extracted prepared with ethanol/water (8/2, by weight ratio).
(the composition ratios are expressed on a dry solid basis.)

The followings are the criterion for skin-whitening effect and the judgement thereof when the test cosmetic solutions and the comparison cosmetic solutions are used.

(Criterion)

Distinctly effective :     Pigmentation became substantially inconspicuous.
Effective :                    Pigmentation became highly pale.
Slightly effective :        Pigmentation became slightly pale.
Ineffective :                 Unchanged.

(Judgement)

A : Among the volunteers, percentage (effective percentage) of distinctly effective or effective is 80% or more.

B : Among the volunteers, percentage (effective percentage) of distinctly effective or effective is not less than 50% but less than 80%.

C : Among the volunteers, percentage (effective percentage) of distinctly effective or effective is not less than 30% but less than 50%.

D : Among the volunteers, percentage (effective percentage) of distinctly effective or effective is less than 30%.

As evident from the result in Table 5, the skin-whitening effect was evaluated as A or B when used the test cosmetic lotions (external dermal agents) prepared by combining the extract powder of indigo plant and any one of L-ascorbic acid 2-glucoside, potassium 4-methoxysalicylate, kojic acid, arbutin, tranexamic acid, ellagic acid, 4-n-butylresorcinol, linoleic acid, chamomile extract, and tetrahydrocurcumin. While the skin-whitening effect was evaluated as B or C when used the comparison cosmetic solutions prepared by only incorporating any one of L-ascorbic acid 2-glucoside, potassium 4-methoxysalicylate, kojic acid, arbutin, tranexamic acid, ellagic acid, 4-n-butylresorcinol, linoleic acid, chamomile extract, and tetrahydrocurcumin; and any one of the other ingredients with skin-whitening effect was bumped down at least a notch compared with those used in combination with the extract powder of indigo plant. This result indicates that the external dermal agent, prepared by incorporating the extract powder of indigo plant and any one of the other ingredients with skin-whitening effect, will exert an improved skin-whitening effect through a synergistic action of the extract powder and any one of the other ingredients.

<Experiment 6: Influence of extract powder of indigo plant of the family *Polygonaceae* on elastase activity>

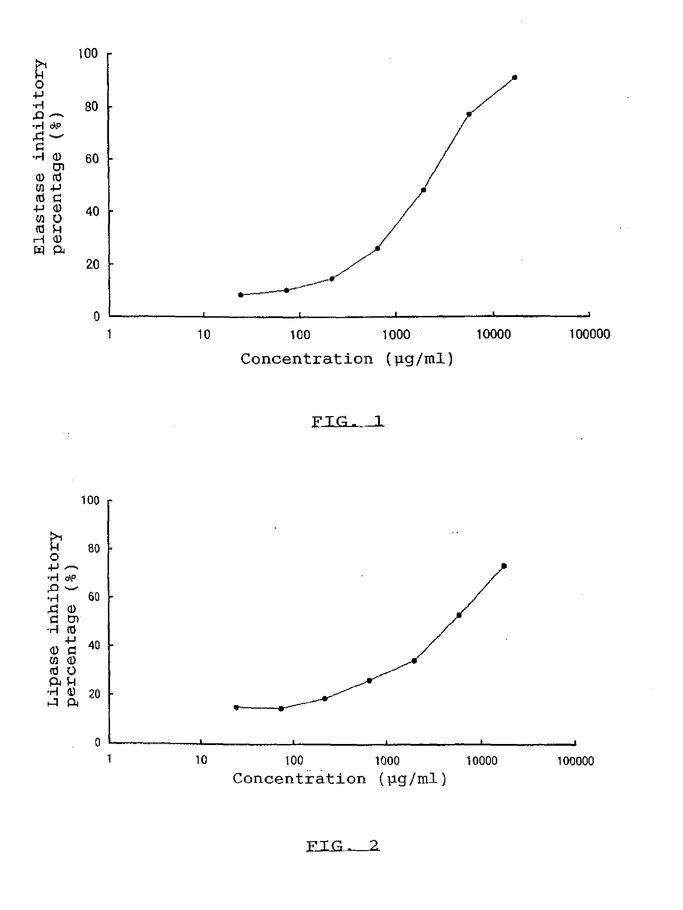

The following experiment was conducted to examine the influence of an extract powder of indigo plant of the family *Polygonaceae* on elastase activity. An extract powder of indigo plant was diluted with refined water to give a concentration of 1.5%, the dilute was sequentially diluted with refined water to obtain a test specimen with a solid content of 15,000 $\mu$g/ml, 5,000 $\mu$g/ml, 1,670 $\mu$g/ml, 560 $\mu$g/ml, 190 $\mu$g/ml, 60 $\mu$g/ml, or 20 $\mu$g/ml. To 50 $\mu$l of any one the test specimens was added 25 $\mu$l of 0.2 M Tris-HCl buffer (pH 8.5, 1% BSA, and 1M-NaCl) containing 20 $\mu$M of a synthetic substrate of N-methoxysuccinyl-L-alanyl-L-alanyl-L-prolyl-L-valine-4-methyl-coumaryl-7-amide (commercialized by Peptide Institute, Inc.), admixed with 25 $\mu$l of 0.2M Tris-HCl buffer containing 0.048 unit/ml of an elastase derived from human neutrophil, and incubated at 37° C for 60 min. Thereafter, the amount of 4-nitroaniline formed as a decomposed product of the enzymatic reaction was measured for fluorescent intensity (Ex 355 nm, Em 460 nm) using "FLUORESCENT MICROPLATE READER FLUOROSCAN II", commercialized by BIO-TEK INSTRUMENT. The inhibitory percentage of elastase activity was calculated by the following equation. The result is in FIG. 1.

$$\text{Inhibition percentage (\%)} = \{(A-B)/A\} \times 100$$

A:   Fluorescent intensity with no addition of test specimen
B:   Fluorescent intensity with addition of test specimen

As shown in FIG. 1, the extract powder of indigo plant was observed to have a concentration-dependent inhibitory activity against elastase activity. Similar experiment was conducted using an aqueous solution prepared by dissolving in water the extract of Japanese knotweed, buckwheat, or knotgrass, used in Experiment 1, to give a concentration of 1.5%, d.s.b., and the concentration of each of the resulting test specimens that inhibits the enzymatic activity of the used elastase by 50% ($IC_{50}$) was compared with that of the test specimen of the extract powder of indigo plant, revealing that the $IC_{50}$ of the test specimen of the extract powder of indigo plant was 1.11 mg/ml, while the test specimens of Japanese knotweed, buckwheat, and knotgrass showed inhibitory percentages against elastase activity at low levels of 12%, 13% and 15%, respectively, and there could not be calculated any concentration that inhibits the elastase activity by 50%. The result indicates that the extract powder of indigo plant has a stronger inhibitory activity against elastase activity than those of Japanese knotweed, buckwheat, and knotgrass, which belong to the same family *Polygonaceae* as indigo plant. Also the result shows that external dermal agents containing the extract powder of indigo plant have a strong effect of inhibiting the generation of wrinkles.

<Experiment 7: Influence of extract powder of indigo plant on lipase activity>

The following experiment was conducted to examine the influence of extract powder of indigo plant on lipase activity. Twenty-five microliters of any one of the solution of the extract powder of indigo plant and the solutions of test specimens prepared by diluting the above solution, which had been used in Experiment 6, and 50 $\mu$l of McIlvaine's buffer solution (pH 7.4, containing 9.15% of 0.1 M citric acid and 90.85% of 0.2 M Na$_2$HPO$_4$) containing 0.1 M 4-methylumbelliferyloleate as a substrate were mixed, admixed with 25 $\mu$l of McIlvaine buffer containing one unit/ml of lipase, and enzymatically reacted at 37° C for 20 min. Thereafter, 50 $\mu$l of 1 N-HCl and 100 $\mu$l of sodium citrate were added to the reaction mixture to suspend the enzymatic reaction, followed by determining the amount of 4-methylumbelliferon formed as a decomposition product of enzymatic reaction by measuring the fluorescent intensity (Ex 355 nm, Em 460 nm) using "FLUORESCENT MICROPLATE READER FLUOROSCAN II", commercialized by BIO-TEK INSTRUMENT. The inhibitory percentage against elastase activity was calculated by the following equation. The result is in FIG. 2.

$$\texttt{Inhibition percentage (\%) = \{1 - (A-B)/(C-B)\} x 100}$$

A:    Fluorescent intensity with addition of test specimen
B:    Fluorescent intensity with only McIlvaine buffer
C:    Fluorescent intensity with no addition of test specimen

As shown in FIG. 2, the extract powder of indigo plant was observed to have a concentration-dependent inhibitory activity against lipase activity. The result indicates that external dermal agents containing the extract powder of indigo plant have an improved anti-acne effect.

<Experiment 8: Influence of extract powder of indigo plant on SOD-like activity>

The following experiment was conducted to examine the influence of extract powder of indigo plant on SOD-like activity. It was tested using "SOD TEST WAKO", a product commercialized by Wako Pure Chemical Industries, Tokyo, Japan, based on the theory of nitroblue tetrazolium (NBT) reduction test.
Ten microliters of any one of the solution of the extract powder of indigo plant and the solutions of test specimens prepared by diluting the above solution, which had been used in Experiment 6, and 95 $\mu$l of a color-developing reagent containing 0.24 M NBT and 0.4 M xanthine, admixed with 95 $\mu$l of an enzyme (xanthine oxidase) solution, and enzymatically reacted at 37° C for 30 min. Thereafter, the enzymatic reaction was suspended with 100 $\mu$l of a reaction-suspending solution (69 mM sodium dodecyl sulfate) and subjected to measurement of absorbance at OD 562 nm using "MICROPLATE READER EL-340", commercialized by BIO-TEK INSTRUMENT. There were provided, as a blank, a sample with addition of refined water in place of the enzyme and, as a control, a sample with addition of a solvent in place of a test specimen, and the extinguishing percentage (%) of superoxide anion (O$^{2-}$) was calculated by the following equation. The result is in FIG. 3.

$$\texttt{Extinguishing percentage (\%) of superoxide anion (O}^{2-}\texttt{)}$$
$$\texttt{= \{(A-B)/A\} x 100}$$

A:    Absorbance for control
B:    Absorbance for the system with no addition of test specimen

As shown in FIG. 3, there was observed a concentration-dependent SOD-like activity (extinguishing activity for superoxide anion). The result shows that external dermal agents containing the extract powder of indigo plant have an improved anti-ageing inhibitory effect.

<Experiment 9: Influence of extract powder of indigo plant on 1,1-diphenyl-2-picryl hydrazyl radical>

An experiment was conducted to examine the influence of an extract powder of indigo plant on 1,1-diphenyl-2-picryl hydrazyl (abbreviated as "DPPH", hereinafter) radical. An extract powder of indigo plant was diluted with 5 mM acetate buffer (pH 5.5) to give a solid concentration of 1.4% and successively diluted by 3-times to obtain a test specimen with a solid concentration of 14,000 μg/ml, 4,700 μg/ml, 1,570 μg/ml, 520 μg/ml, 170 μg/ml, 58 μg/ml, or 19 μg/ml. Fifty microliters of any one of the test specimens was mixed with 100 μl of 1 mM DPPH ethanol solution, stirred, allowed to react at ambient temperature for 20 min, and measured for absorbance at 515 nm using "MICROPLATE READER EL-340", commercialized by BIO-TEK INSTRUMENT. The degree of activity was expressed with DPPH radical elimination percentage (%) calculated by the following equation. The result is in FIG. 4.

```
DPPH radical elimination percentage (%) = {1-(A-B)/(C-B)} x 100
```

A:    Absorbance with addition of test specimen
B:    Absorbance with only acetate buffer
C:    Absorbance for the case free of test specimen

As shown in FIG. 4, there was observed that the extract powder of indigo plant has a concentration-dependent DPPH radical eliminating activity. The result shows that external dermal agents containing the extract powder of indigo plant have an improved anti-ageing effect.

<Experiment 10: Influence of extract powder of indigo plant on skin inflammatory>

As evident from Experiments 6 to 9, the extract powder of indigo plant has been proved to have a skin-whitening effect and further to have effects of inhibiting the activity of enzymes that are recognized to influence on ageing and the generation of wrinkles in the skin, and thus the influence of the extract powder on the skin was confirmed by a volunteer test. Test gels were prepared by providing 35 parts by weight of a 2% aqueous carboxyvinylpolymer solution, 5 parts by weight of concentrated glycerin, 1.8 parts by weight of a 10% aqueous potassium hydroxide solution, and 3 parts by weight of pentylene glycol, and an adequate amount of a solution of an extract powder of indigo plant which had had been prepared in accordance with the method in the later described Example 1, which had been prepared by dissolving the extract powder in refined water to give a concentration of 1% of indigo extract, d.s.b., and mixing the above with an appropriate amount of refined water to adjust the concentration of the indigo plant extract to any one of those listed in Table 6 to be incorporated into gels. As a control, a control gel was prepared by using a 1% aqueous dextrin solution in place of 50 parts by weight of the solution of extract powder of indigo plant having the above composition. Sixty volunteers (subjects) were divided into six groups consisting of 10 volunteers each, and to 10 volunteers in each group were applied with any one of the test gels, which contained any one of the aqueous solutions with different concentrations, and the control gel at a different part of the internal part of the upper arm of each subject three times a day for successive 56 days. On the day after the final application, each volunteer was irradiated with ultraviolet ray at a dose of 2-times of a minimal erythema dose (MED), i.e, a minimal irradiation dose that induces erythema at an irradiated part, which had been confirmed on an irradiation test by varying the irradiation dose of ultraviolet ray, at a part applied with any one of the test gels and the control gel. At 24 hours after the ultraviolet irradiation, the volunteers were observed their parts applied with any one of the test gels and the control gel to confirm the degree of occurrence of inflammation (erythema) macroscopically and the degree of changing of elasticity of the skin by palpation. The followings are the criterion and the judgement of the effects by the gels, and the result in Table 6.

(Criterion)

<Erythema>

Distinctly effective    : Occurrence of erythema was not substantially observed in the part applied with a test gel.

Effective    : Occurrence of erythema was clearly inhibited compared with the part applied with the control gel.

Ineffective    : Substantially the same level of erythema was occurred as the part applied with the control gel.

Aggravated    : Occurrence of erythema was aggravated compared with the part applied with the control gel.

<Elasticity>

Strongly effective : Improved elasticity and increased skin thickness were found in the part applied with a test gel.

Effective : Improved elasticity was found in the part applied with a test gel but not found any difference in skin thickness.

Ineffectiveness : No difference was found compared with the part applied with the control gel.

Aggravated : Elasticity was lowered compared with the part applied with the control gel.

(Judgement)

A : Among the volunteers, percentage (effective percentage) of distinctly effective or effective is 80% or more.

B : Among the volunteers, percentage (effective percentage) of distinctly effective plus effective is not less than 50% but less than 80%.

C : Among the volunteers, percentage (effective percentage) of distinctly effective plus effective is not less than 30% but less than 50%.

D : Among the volunteers, percentage (effective percentage) of distinctly effective plus effective is less than 30%.

[Table 6]

| Composition ratio of extract powder of indigo plant (concentration of solids in indigo extract) (%) | Judgement | |
|---|---|---|
| | Degree of erythema | Elasticity of the skin |
| 0.5 | A | A |
| 0.05 | A | A |
| 0.005 | A | B |
| 0.0005 | B | B |
| 0.0001 | C | C |

As evident from Table 6, the occurrence of erythema in the skin, applied with the test gels incorporated with 0.0005% of the extract powder of indigo plant in terms of the dry solids of indigo plant extract, was evaluated as B compared with the one applied with the control gel; the occurrence of erythema and the elasticity in the skin, applied with the test gels incorporated with 0.005% or more of the extract powder of indigo plant were both evaluated as B, where a satisfactory effect was observed due to the application of the test gels in at least half the number of the volunteers; the effect became more apparent when the test gels were incorporated with 0.005% or more of the extract powder; and the application effect became more distinct such that the inhibition of occurrence of erythema and the elasticity in the skin were both evaluated as A when the test gels, incorporated with 0.05% or more of the extract powder, were used.

<Experiment 11: Influence of extract powder of indigo plant on the skin inflammation>

As evident from the result in Experiment 10, the gels, incorporated with at least 0.0005% of the extract powder of indigo plant in terms of the dry solids of indigo plant extract, inhibit the occurrence of inflammation (erythema) in the skin induced by ultraviolet ray and distinctly augment the elasticity of the skin, and then it was conducted histological examination of the skin applied with an aqueous solution of extract powder of indigo plant. A test gel was prepared by mixing 35 parts by weight of a 2% aqueous solution of carboxyvinylpolymer, five parts by weight of concentrated glycerin, 1.8 parts by weight of a 10% aqueous solution of potassium hydroxide, three parts by weight of pentylene glycol, 50 parts by weight of an aqueous solution of an extract powder of indigo plant, which had had been prepared in accordance with the method in the later described Example 1, that had been prepared by adding refined water to the extract powder to give a solid concentration of one percent, and 5.2 parts by weight of refined water. As a control, a control gel having the same composition as the test gel was similarly prepared as in the above except for adding thereto 50 parts by weight of "DEXYPEARL SD-20, DE 1.4", a saccharide containing one percent of cyclodextrin commercialized by Ensuiko Sugar Refining Co., Ltd., Tokyo, Japan, in place of 50 parts by weight of the above aqueous solution of extract powder of indigo plant. The test gel or the control gel was applied to a different internal part of the upper arm of each of five volunteers (subjects) three times a day for successive 56 days. On the final application day, similarly as in Experiment 10, the part of each volunteer, to which had been applied with the test gel or the control gel, was irradiated with ultraviolet ray at a double dose of the minimal erythema dose (MED) to each volunteer. At 24 hours after the irradiation, a skin tissue in the part in the internal upper arm of each volunteer anesthetized with lidocaine, which had been irradiated with ultraviolet ray and applied with either the test gel or the control gel, or that in the part free of any application was sterilely collected using a commercialized biopsy punch. The collected tissues were in usual manner fixed with formalin, embedded in paraffin, made into sectioned tissues, and histologically examined. The sectioned tissues were used for measurement of the thickness of the epidermal layer and the dermal layer, the number of sunburned cells (death cells) induced by the ultraviolet ray irradiation, and the observation of the form of elastic fiber in the dermis by Elastica-van Gieson method. The thickness of the epidermal and dermal layers was determined by randomly selecting 30 sites in each sectioned tissue under a light microscope, photographing the selected sites by a digital camera, measuring the thickness of the desired part in each photograph using a slide calipers, and averaging the data. Each of the obtained mean values for the thickness of the sectioned tissue applied with the test gel or the control gel was divided by the mean value for that applied with no gel to obtain an increased percentage (%) of the thickness of the epidermal or dermal layer. Increased percentages (%) of five volunteers were averaged and the result is shown in Table 7 in parallel. Similarly, the number of sunburned cells was counted by photographing randomly selected 30 sites in each sectioned tissue under a light microscope, counting the sunburned cells observed in each site, and averaging the data to obtain a mean value. The mean value of the sunburned cells in the sites applied with the test gel or the control gel was divided by that applied with no gel to obtain an increased percentage (%) of sunburned cells. Increased percentages (%) of five volunteers were averaged and the result is shown in Table 7 in parallel.

[Table 7]

| Type of gel applied | Items measured | | |
| --- | --- | --- | --- |
| | Increased percentage of epidermal layer (%) | Increased percentage of dermal layer (%) | Increased percentage of sunburned cells (%) |
| Test gel | 141 | 143 | 116 |
| Control gel | 118 | 120 | 149 |

As evident from Table 7, compared with the sites applied with no gel, the sites applied with the test gel or the control gel were increased in thickness of the epidermal and dermal layers and also increased in number of sunburned cells. The increased percentages (%) in the epidermal and dermal layers of the sites applied with the test gel were higher than those applied with the control gel, while the increased percentage (%) of sunburned cells for the test gel was lower than that for the control gel. Upon observation of elastic fiber under a light microscope, the site applied with no gel had regularly arranged fibers, but that applied with the control gel had an irregularly arranged fiber. While in the site applied with the test gel, it was observed an increment of regularly-arranged elastic fibers throughout the dermal layer. These data indicate that the incorporation of the extract powder of indigo plant into external dermal agents for use will increase the turnover of cells in the epidermal and dermal layers, augment the elasticity of the skin, and protect the skin cells from ultraviolet ray hazard, and thus the extract powder of indigo plant is useful as an anti-ageing agent.

<Experiment 12: Influence of extract powder of indigo plant on body fats in humans>

The following experiment was conducted to examine the influence of an extract powder of indigo plant on body fats in humans. A milky lotion for test (abbreviated as "test lotion", hereinafter) was prepared in usual manner by adding refined water to 0.5 part by weight of an extract powder of indigo plant, one part by weight of stearic acid, two parts by weight of cetanol, 2.5 parts by weight of petrolatum, 4.0 parts by weight of squalane, one part by weight of L-alginic acid, one part by weight of glyceryl monostearate lipophilic, one part by weight of glycerin, 0.1 part by weight of potassium hydroxide, an adequate amount of a flavor, and refined water to give a total amount of 100 parts by weight. Twenty male and female volunteers (consisting of 10 males and 10 females) as subjects were applied with the test lotion to their right upper arms two times a day, and measured for the skinfold thickness in the parts applied with the test lotion by using "UX-1", a product name of ultrasonograph commercialized by Orion Co., Ltd., Tokyo, Japan, at the initiation of the application (zero week) and successive every week (four weeks in total), followed by calculating the reduction rate of panniculus adiposus [100-{(weekly skinfold thickness) x 100 / (skinfold thickness at the initiation of the test)}] based on the ratio of respective data to that of the initiation application [(weekly skinfold thickness) / (the skinfold thickness at the initiation of the test)]. As a control, a control milky lotion (abbreviated as "control lotion", hereinafter) having the same composition as the test lotion was similarly prepared as in the above except for incorporating 0.5 part by weight of "DEXYPEARL SD-20, DE 1.4", a saccharide containing one percent of cyclodextrin commercialized by Ensuiko Sugar Refining Co., Ltd., Tokyo, Japan, in place of the extract powder of indigo plant, and applied to the left upper arm of each of the same volunteers as in case of using the test lotion, followed by measuring their skinfold thickness. The reduction rates of panniculus adiposus in eight volunteers, which had been applied with the test lotion or the control lotion, were averaged, and the slimming effects of the lotions were evaluated based on the following four ranks. The results and the mean values of the reduction rates of panniculus adiposus in the eight volunteers are shown in Table 8 in parallel. Reduction rates of panniculus adiposus:

0 to 5%, Inefficacious (-)
6 to 10%, Slightly efficacious ($\pm$)
11 to 15%, Efficacious, (+)
16% or more, Distinctly efficacious (++)

[Table 8]

| Lotion type | Initiation (0 week) | 1 Week | 2 Week | 3 Week | 4 Week |
|---|---|---|---|---|---|
| Test lotion | 0%<br>(-) | 10%<br>($\pm$) | 12%<br>(+) | 21%<br>(++) | 28%<br>(++) |
| Control lotion | 0%<br>(-) | 0%<br>(-) | 0%<br>(-) | 0%<br>(-) | 0%<br>(-) |

As evident from Table 8, when applied with the test lotion, it was observed efficacy after 2-week application and observed distinct efficacy after 3-week or more application. While, when applied with the control lotion, no distinct changing was found in the skinfold thickness throughout the experiment. The result indicates that the extract powder of indigo plant is useful as a slimming agent.

<Experiment 13: Influence of extract powder of indigo plant on microorganisms in human oral cavity>

The following experiment was conducted to examine the influence of an extract powder of indigo plant on microorganisms in human oral cavity. An extract powder of indigo plant, prepared by the method in the later described Example 1, was dissolved in refined water to obtain an aqueous solution with a concentration of 1.5% indigo plant extract, d.s.b. Using the aqueous solution, the minimum inhibitory concentration (may be designated as "MIC", hereinafter) against microorganisms in human oral cavity (microorganisms as causatives of periodontal diseases and dental caries) was determined. Tryptanthrin, gallic acid, and caffeic acid, which had been confirmed to be contained in the indigo extract, were determined for MIC against microorganisms in human oral cavity, and the data was shown in Table 9 in parallel. The following method was used for assaying MIC.

<Assay for minimum inhibitory concentration (MIC)>

The MICs of an extract powder of indigo plant, tryptanthrin, gallic acid, and caffeic acid against microorganisms in human oral cavity were determined according to the standard method of Japanese Society of Chemotherapy (see "Saikin-Shinkin-Kensa (Test for Bacteria and Fungi), 2nd Edition", pp. 524-529 (1982)). Examples of microorganisms, used as causatives of periodontal diseases, were microorganisms of *Prophyromonas gingivalis* strains such as JCM8525, JCM12257, and ATCC 33277 strains; those of *Actinobacillus actinomycetemcomitans* (may be abbreviated as "A. *actinomycetemcomitans*") such as JCM2434 and JCM8577 strains; *Prevotella intermedia* (may be abbreviated as *P. intermedia*) JCM7365 strain; and *Campylobacter rectus* (may be abbreviated as *C. rectus*) JMC6301 strain. Examples of microorganisms, used as causatives of dental caries, were *Streptococcus mutans* strains such as OMZ-175, OMZ-176, OMZ-65, OMZ-61, B-13D, and Ingbritt strains; and *Streptococcus sobrinus* (may be abbreviated as "*S. sobrznus*") 6715 strain. Among these microorganisms, those which are causatives of periodontal diseases were cultured at 37° C for 48 hours, while those which are causatives of dental caries were cultured at 37° C for 18 to 24 hours under anaerobic conditions by using a brain heart infusion (abbreviated as "BHI", hereinafter), commercialized by Difco Laboratories, Detroit, USA, supplemented with 5 μg/ml of hemin, and 0.5 μg/ml of vitamin K$_3$. The resulting cultures of the microorganisms, as causatives of both periodontal diseases and dental caries, were respectively diluted into solutions with 10$^8$ cells and 10$^6$ cells. Five microliters of each of which was inoculated to a BHI agar plate, which had been admixed with the aqueous solution of extract powder of indigo extract, tryptanthrin, gallic acid, or caffeic acid intact or after diluted to give different concentrations, and cultured at 37° C for 24 hours while keeping anaerobic conditions, followed by determining the inhibitory concentrations (MIC) of the above ingredients that completely inhibit the growth of the microorganisms.

Table 9

| Microorganisms in oral cavity | | Minimum inhibitory concentration (MIC) | | | |
|---|---|---|---|---|---|
| | | Indigo extract (mg/ml) | Tryptanthrin | Gallic acid | Caffeic acid |
| | | | (μg/ml) | | |
| P. gingivalis | JMC12257 | 1.74 | 12.5 | 400 | 400 |
| | JMC8525 | 1.74 | 6.25 | 400 | 400 |
| | JMC33277 | 1.74 | 12.5 | 400 | 400 |
| A. actinomycetemcomitans | JMC2434 | 3.84 | 25 | 800 | 400 |
| | JMC8577 | 3.84 | 25 | 800 | 200 |
| C. rectus | JMC6301 | 1.74 | 25 | 400 | 100 |
| P. intermedia | JMC7365 | 1.74 | 6.25 | 400 | 400 |
| S. mutans | OMZ-176 | 3.84 | 12.5 | 400 | 800 |
| | OMZ-175 | 3.84 | 6.25 | 400 | 400 |
| | OMZ-65 | 3.84 | 12.5 | 1,600 | 200 |
| | B-13D | 1.74 | 6.25 | 200 | 200 |
| | Ingbritt | 3.84 | 25 | 1,600 | 1,600 |
| | OMZ-61 | 1.74 | 6.25 | 200 | 400 |
| S. sobrinus | 6715 | 3.84 | 25 | 800 | 800 |

As evident from Table 9, the extract powder of indigo plant showed a relatively strong antibacterial activity against the microorganisms of the four species and seven strains which are causatives of periodontal diseases, and those of two species and seven strains which are causatives of dental caries. The MICs were 1.74 to 3.48 mg/ml, d.s.b., in terms of the indigo plant extract in the extract powder of indigo plant. While tryptanthrin was observed to have a relatively strong antibacterial activity and both gallic acid and caffeic acid had a relatively weak antibacterial activity, and their MICs were respectively 6.25 to 25 μg/ml, 200 to 1,600 μg/ml, and 200 to 1,600 μg/ml. Considering the extract powder of indigo plant used in this experiment contained tryptanthrin, gallic acid, or caffeic acid in respective amounts of about 0.51 μg/mg, about 0.55 μg/mg, or 1.07 μg/mg, which could not be sufficient to achieve the MICs against periodontal diseases and dental caries, suggesting that there exists any ingredient(s) in the extract powder, other than tryptanthrin, gallic acid, and caffeic acid, that would correlate to the antibacterial activity against periodontal diseases and dental caries. The inhibitory effect of each ingredient on the growth of respective microorganisms used in the assay for MIC was observed from a concentration of about 1/100 of MIC of each ingredient, and the effect increased in a dose dependent manner.

<Bactericidal action>

The following experiment was conducted to confirm the bactericidal action of an extract powder of indigo plant against Prophyromonas gingivalis JCM8525 strain used for assaying MIC. A fresh preparation of the same extract powder of indigo plant as used in the assay for MIC was added to BHI to give a concentration of 6.95 mg/ml, 3.84 mg/ml, or 1.74 mg/ml, d.s.b., of the indigo plant extract, inoculated with Prophyromonas gingivalis JCM8525 strain to give a viable cell number of 10$^8$ cells/ml, and cultured at 37° C for three or nine hours while keeping anaerobic conditions. The resulting cultures were appropriately diluted, inoculated to a BHI agar plate, and subjected to culturing at 37° C for 24 hours while keeping anaerobic conditions, followed by counting the formed colonies and calculated the number of viable cells in each culture before dilution. As a control, the number of viable cells after culturing based on that obtained after culturing in the BHI plate to which only an aqueous solution containing "DEXYPEARL SD-20, DE 1.4", a saccharide containing one percent of cyclodextrin commercialized by Ensuiko Sugar Refining Co., Ltd., Tokyo, Japan, had been added in place of the aqueous solution of the extract powder of indigo plant.

When cultured for three hours in a medium supplemented with the aqueous solution containing 6.95 mg/ml, d.s.b., of the extract powder of indigo plant, *Prophyromonas gingivalis* JCM8525 strain was completely sterilized. When cultured in a BHI medium supplemented with an aqueous solution containing 3.48 mg/ml of the extract powder, the viable cells were reduced to 1/1,000 after 3-hour culturing and to 1/10,000 after 9-hour culturing compared to that before culturing. When cultured in a BHI medium supplemented with 1.74 mg/ml of the aqueous solution of the extract powder, the viable cells were reduced to about 1/10 after 3-hour culturing and to about 1/100 after 9-hour culturing compared to that before culturing. The viable cells of *Prophyromonas gingivalis* JCM 8525 strain were reduced depending on both the solid concentration of the indigo plant extract in the extract powder of indigo plant and the culturing time after admixed with the aqueous solution of the extract powder of indigo plant. While, when cultured in a BHI medium incorporated with only the aqueous solution containing only saccharides free of the extract powder of indigo plant, no reduction of the number of viable cells was observed, confirming that the aqueous solution of the extract powder of indigo plant shows a relatively strong bactericidal action against the microorganisms of *Prophyromonas gingivalis*.

These results indicate that the extract powder of indigo plant of the present invention has all the following respective activities of melanin formation inhibitory action, elastase activity inhibitory action, lipase activity inhibitory action, active-oxygen-eliminating action, radial-entrapping action, turnover-promoting action on epidermal and dermal cells, action of protecting cells from ultraviolet ray hazard, and action of lowering body fats; the external dermal agents incorporated with the extract powder of indigo plant will inhibit spots, freckles, and pigmentation after sunburn, inhibit the formation of wrinkles and fine wrinkles, reduce panniculus adiposus, and inhibit the ageing of the skin, and therefore the agents have an improved skin-whitening effect, slimming action, skin-beautifying action, and/or anti-ageing action. They also indicate that the extract powder of indigo plant has a satisfactory effect of preventing and improving periodontal diseases and dental caries, when used in orally ingestible products and orally-usable external dermal agents.

The present invention is explained with reference to the following Examples in more detail but they should never restrict the present invention:

Example 1

<Preparation of extract powder of indigo plant>

Thirty kilograms of water was placed in a jacketed tank, heated to 90° C with steam, and admixed with 2.5 kg of a preparation prepared by finely cutting dried leaves of *Polygonum tinctorium* Lour. with a mill, followed by extracting the cut leaves with steam for 60 min. The resultant was in usual manner centrifuged with a filter fabric in combination with high-speed centrifugation to remove residues, resulting in an obtention of 20 kg of a liquid extract of indigo plant. The extract was filtered with "Ultrafiltration Module MICROZA API2013", a UF membrane commercialized by Asahikasei Co., Ltd., Tokyo, Japan, heated under a reduced pressure to evaporate moisture to obtain six kilograms of a concentrated solution. The solution was measured for solid content and to one part by weight of which was admixed with one part by weight, d.s.b., of "SANDEC #30" with a DE 3.5, a product name of a dextrin produced by Sanwa Cornstarch Co., Ltd., Nara, Japan, followed by spray-drying the mixture in usual manner to obtain 0.6 kg of an extract powder of indigo plant. The product is a readily handleable powder which neither absorbs moisture nor solidifies even when stored under a relative humidity of 53% at 25° C for one week or more. The product contained 52 μg of tryptanthrin and 28 mg of polyphenol per one gram thereof. The product can be tabletted alone or incorporated into compositions to produce foods, cosmetics, quasi-drugs, pharmaceuticals, feeds, baits, pet foods, etc.

Since the product has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, therapeutic action on dermatophytosis, therapeutic action on stomatitis, therapeutic action on acne, and inhibitory action on periodontal diseases and dental caries, it can be arbitrarily used as an effective ingredient in orally-ingestible compositions including external dermal agents and food products for the purpose of exerting slimming effect, anti-ageing action, skin-whitening, and/or skin-beautifying.

Example 2

<Preparation of extract powder of indigo plant>

Using 10 kg of an indigo liquid extract after filtration with a UF-membrane, prepared by the same method as in Example 1, an about 200 g of an extract powder of indigo plant was prepared by admixing one part by weight, d.s.b., of "DEXYPEARL SD-20, DE 1.4", a saccharide containing a branched cyclodextrin commercialized by Ensuiko Sugar Refining Co., Ltd., Tokyo, Japan, with one part by weight of the solid content of the indigo liquid extract and, in usual manner, spray drying the resulting mixture. The product is a readily handleable powder which neither absorbs moisture nor solidifies even when stored under a relative humidity of 53% at 25° C for one week or more. The product contained 50 μg of tryptanthrin and 27 mg of polyphenol per one gram thereof. The product can be tabletted alone or incorporated into compositions to produce foods, cosmetics, quasi-drugs, pharmaceuticals, feeds, baits, pet foods, etc.

Since the product has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, therapeutic action on dermatophytosis, therapeutic action on stomatitis, therapeutic action on acne, and inhibitory action on periodontal diseases and dental caries, it can be arbitrarily used as an effective ingredient in orally-ingestible compositions including external dermal agents and food products for the purpose of exerting slimming effect, anti-ageing action, skin-whitening, and/or skin-beautifying.

Example 3

<Preparation of extract powder of indigo plant>

Whole plant of a raw Japanese indigo plant *Polygonum* tinctorium Lour. was finely cut, and four parts by weight of which was mixed with three parts by weight of a mixture consisting of refined water and ethanol in an equal weight ratio, and stirred for 20 min for extraction, followed by centrifugation to obtain a supernatant and filtering it with a UF-membrane to obtain an indigo plant extract. The extract thus obtained was subjected to 5-time strength, and to one part by weight of the solid content of the concentrate was added two parts by weight, d.s.b., of "PINEDEX", with a DE of 7.5, a product name of dextrin produced by Sanwa Cornstarch Co., Ltd., Nara, Japan, followed by mixing and dissolving the mixture, freeze-drying the resulting solution, and pulverizing the freeze-dried product to obtain an extract powder of indigo plant. The product is a readily handleable powder which neither absorbs moisture nor solidifies even when stored under a relative humidity of 53% at 25° C for one week or more. The product contained 50 μg of tryptanthrin and 30 mg of polyphenol per one gram thereof. The product can be tabletted alone or incorporated into compositions to produce foods, cosmetics, quasi-drugs, pharmaceuticals, feeds, baits, pet foods, etc.

Since the product has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, therapeutic action on dermatophytosis, therapeutic action on stomatitis, therapeutic action on acne, and inhibitory action on periodontal diseases and dental caries, it can be arbitrarily used as an effective ingredient in orally-ingestible compositions including external dermal agents and food products for the purpose of exerting slimming effect, anti-ageing action, skin-whitening and/or skin-beautifying.

Example 4

<Preparation of extract powder of indigo plant>

An extract powder of indigo plant was prepared similarly as the method in Example 1 except for using a dried leaf of *Isatis tinctoria* L. var yezoensis (a plant of the family *Cruciferae*). The product is a readily handleable powder which neither absorbs moisture nor solidifies even when stored under a relative humidity of 53% at 25° C for one week or more. The product contained 34 μg of tryptanthrin and 19 mg of polyphenol per one gram thereof. The product can be tabletted alone or incorporated into compositions to produce foods, cosmetics, quasi-drugs, pharmaceuticals, feeds, baits, pet foods, etc.

Since the product has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, therapeutic action on dermatophytosis, therapeutic action on stomatitis, therapeutic action on acne, and inhibitory action on periodontal diseases and dental caries, it can be arbitrarily used as an effective ingredient in orally-ingestible compositions including slimming agents and food products for the purpose of exerting anti-ageing action, skin-whitening and/or skin-beautifying, and preventing/alleviating obesity, as well as in external dermal agents for oral use.

Example 5

<Preparation of extract powder of indigo plant>

Using the indigo liquid extract after filtration with a UF-membrane, prepared in Example 1, an extract powder of indigo plant was prepared by adding one part by weight, d.s.b., of "ISOELITE P", a product name of a branched cyclodextrin commercialized by Ensuiko Sugar Refining Co., Ltd., Tokyo, Japan, and 0.1 part by weight of $\alpha,\alpha$-trehalose

to one part by weight of the solid content of the above-identified indigo liquid extract, dissolving the resulting mixture by stirring, and then in usual manner freeze-drying the solution and pulverizing the freeze-dried product. The product is an indigo plant extract that does not cause browning even when stored for a relatively long period of time, has an improved storage stability, and has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, anti-periodontal disease action, and body-fat-reducing action, and thus it can be arbitrarily used as an effective ingredient in slimming agents and external dermal agents for oral use for the purpose of exerting anti-ageing effect, skin-whitening and/or skin-beautifying, and preventing/alleviating obesity.

Example 6

<Preparation of extract powder of indigo plant>

An indigo liquid extract prepared by the method in Example 3 was subjected to 10-time strength, and one part by weight of the solid content of the concentrate was admixed with one part by weight of "ISOELITE P", with a DE of 8, a product name of a branched cyclodextrin commercialized by Ensuiko Sugar Refining Co., Ltd., Tokyo, Japan, and 0.1 part by weight of "TORNARE®", a product name of a syrup containing saccharide derivatives of $\alpha,\alpha$-trehalose, commercialized by Hayashibara Biochemical Laboratories, Inc., Okayama, Japan, followed by dissolving the mixture while stirring and spray drying the resulting solution to obtain an extract powder of indigo plant. The product is an extract powder of indigo plant that does not cause browning even when stored for a relatively long period of time, has an improved storage stability, and has various physiological activities such as melanin formation inhibition, elastase inhibitory activity, lipase inhibitory activity, secretion regulatory action on sebum secreted from sebaceous crypt, SOD-like action, DPPH radical-entrapping action, anti-ageing action, anti-periodontal disease action, and body-fat-reducing action, and thus it can be arbitrarily used as an effective ingredient in slimming agents and external dermal agents for oral use for the purpose of exerting anti-ageing effect, skin-whitening and/or skin-beautifying, and preventing/alleviating obesity.

Example 7

<Cream>

A cream was prepared in usual manner based on the following prescription.

| <Prescription> | (%) |
|---|---|
| Polyglyceryl-10 myristate | 3 |
| "$\alpha$-GLUCOSYL HESPERIDIN", a product name of glucosyl hesperidin commercialized by Hayashibara Biochemical Laboratories, Inc., Okayama, Japan | 1 |
| Lanoline | 0.5 |
| 2-Octyldodecanol | 2 |
| Cetyl 2-ethyl hexanoate | 3 |
| Squalane | 5 |
| Dimethycone | 0.3 |
| Behenyl alcohol | 3 |
| Cetyl alcohol | 2 |
| Butyl alcohol | 1 |
| Cetyl palmitate | 1.5 |
| Glyceryl stearate | 2.3 |
| Butylene glycol | 5 |
| Pentylene glycol | 3.5 |
| Stearic acid | 0.5 |
| Glycerin | 5 |
| "TORNARE®", a product name of a saccharide containing derivatives of $\alpha,\alpha$-trehalose commercialized by Hayashibara Biochemical Laboratories Inc., Okayama, Japan | 2 |
| L-Ascorbic acid 2-glucoside | 2 |
| Pullulan | 0.1 |
| Extract powder of indigo plant prepared by the method in Example 1 | 1 |

(continued)

| <Prescription> | (%) |
|---|---|
| Water | 59.3 |

The product is useful as an external dermal agent for skin-whitening, skin-beautifying, slimming, and/or anti-ageing. Since the product has an improved permeability to and extensibility over the skin and contains pullulan, it has a characteristic of imparting a satisfactory smoothness and elasticity to the skin after application.

Example 8

<Gel>

A gel was prepared in usual manner based on the following prescription.

| <Prescription> | (%) |
|---|---|
| Extract powder of indigo plant prepared by the method in Example 2 | 2 |
| Hyaluronic acid | 0.25 |
| Ellagic acid | 0.6 |
| Kankoso 201 | 0.005 |
| "MINERAL TOREHA", a product name of bittern ingredients commercialized by H + B Life Science Okayama, Japan | 1 |
| $\alpha,\alpha$-Trehalose | 4.5 |
| Glycerine | 5 |
| 1,3-BG | 5 |
| Ethanol | 5 |
| "HIVISWAKO 104", a product name of carboxyvinylpolymer commercialized by Wako Pure Chemical Industries, Tokyo, Japan | 0.64 |
| Polyoxyethylene (20) sorbitan monooleate | 1 |
| 2,2',2"-Nitrotriethanol | 1 |
| Ethyl *p*-hydroxybenzoate | 0.1 |
| Refined water | 74.9 |

The product is useful as an external dermal agent for skin-whitening, skin-beautifying, slimming, and/or anti-ageing. Also the product has an improved permeability to and extensibility over the skin.
When the product was applied to 11 subjects suffering from face inflammation induced by sunburn two times every in the morning and evening, the period required for ameliorating or diminishing such symptom was shortened by 22% on an average compared with that required for other eleven subjects applied with another cream with the same composition as in the above one with no extract powder of indigo plant. When subjects were applied with the product, the degree of skin blackening and the degree of spot occurrence in them, after diminishing inflammation, were lower.

Example 9

<Milky lotion>

A milky lotion was prepared in usual manner based on the following prescription.

| <Prescription> | (%) |
|---|---|
| Polyoxyethylene (20 E.O.) polyoxypropylene (2 E.O.) cetyl alcohol | 1 |
| Silicon KF96 (20 cs) commercialized by Shin-Etsu Chemical Co., Ltd., Tokyo, Japan | 2 |
| Liquid paraffin (with medium viscosity) | 3 |
| 1,3-BG | 5 |
| 4-n-Butyl resorcinol | 0.3 |

(continued)

| <Prescription> | (%) |
|---|---|
| Glycerin | 2 |
| Ethyl alcohol | 15 |
| Carboxyvinyl polymer | 0.3 |
| Kojic acid | 0.5 |
| Hydroxypropylcellulose | 0.1 |
| 2-Aminomethylpropanol | 0.1 |
| Extract powder of indigo plant prepared by the method in Example 2 | 1 |
| Antiseptic | q.s. |
| Refined water | an amount sufficient to adjust the total amount to 100% |

The product is useful as an external dermal agent for skin-whitening, skin-beautifying, slimming, and/or anti-ageing. Also the product has an improved permeability to and extensibility over the skin.

Example 10

<Milky lotion>

A milky lotion was prepared in usual manner based on the following prescription.

| <Prescription> | (%) |
|---|---|
| Polyoxyethylene (20 E.O.) polyoxypropylene (2 E.O.) cetyl alcohol | 1 |
| Silicon KF96 (20 cs) commercialized by Shin-Etsu Chemical Co., Ltd., Tokyo, Japan | 2 |
| Liquid paraffin (with medium viscosity) | 3 |
| 1,3-BG | 5 |
| L-Ascorbic acid 2-glucoside | 2 |
| Glycerin | 2 |
| Ethyl alcohol | 15 |
| Carboxyvinyl polymer | 0.3 |
| Hydroxypropylcellulose | 0.1 |
| 2-Aminomethylpropanol | 0.1 |
| Extract powder of indigo plant prepared by the method in Example 2 | 0.1 |
| Antiseptic | q.s. |
| Refined water | an amount sufficient to adjust the total amount to 100% |

The product is useful as an external dermal agent for skin-whitening, skin-beautifying, slimming, and/or anti-ageing. Also the product has an improved permeability to and extensibility over the skin.

Example 11

<Milky lotion>

A milky lotion was prepared in usual manner based on the following prescription.

| <Prescription> | (%) |
|---|---|
| Extract powder of indigo plant prepared by the method in Example 2 | 1 |
| Co-Lipase | 0.1 |
| Stearic acid | 1 |

(continued)

| <Prescription> | (%) |
|---|---|
| Cetanol | 2 |
| Arbutin | 1 |
| Petrolatum | 2.5 |
| Squalane | 4 |
| L-Arginine | 1 |
| "α-GLUCOSYL HESPERIDIN", a product name of glucosyl hesperidin commercialized by Hayashibara Biochemical Laboratories, Inc., Okayama, Japan | 1 |
| Glyceryl monostearate, lipophilic | 1 |
| Glycerin | 2 |
| Potassium hydroxide | 0.1 |
| Flavor | q.s. |
| Refined water | an amount sufficient to adjust the total amount to 100% |

The product is useful as an external dermal agent for skin-whitening, skin-beautifying, slimming, and/or anti-ageing. Also the product has an improved permeability to and extensibility over the skin.

Example 12

<Shampoo>

A shampoo was prepared in usual manner based on the following prescription.

| <Prescription> | (%) |
|---|---|
| Piroctone olamine | 0.5 |
| Disodium edetate | 0.3 |
| Kankoso 210 | 0.002 |
| Citric acid | 0.3 |
| Sodium salicylic acid | 0.2 |
| 1,3-BG | 3 |
| Sodium polyoxyethylene laurylether sulfate | 6.75 |
| Coconut fatty acid diethanolamide | 2 |
| Triethanolamine lauryl sulfate | 10 |
| Polyoxyethylene lanolic acid (80 E.O.) | 0.5 |
| 2-Alkyl-N-carboxymethyl-N-hydroxyethyl imidazolinium betaine | 10 |
| O-[2-Hydroxy-3-(triethylammonio)propyl] hydroxyethylcellulose chloride | 0.8 |
| Extract powder of indigo plant prepared by the method in Example 2 | 1 |
| "α-GLUCOSYL HESPERIDIN", a product name of glucosyl hesperidin commercialized by Hayashibara Biochemical Laboratories, Inc., Okayama, Japan | 1 |
| Flavor | 0.2 |
| Refined water | an amount sufficient to adjust the total amount to 100% |

Since the product is a shampoo which has an effect of preventing both inflammation in the scalp and ageing and has a relatively strong antibacterial activity, it exerts an improved hair growth effect, inhibits loss of hair, and keeps the scalp clean.

Example 13

<Hair tonic>

A hair tonic was prepared in usual manner based on the following prescription.

| <Prescription> | (%) |
| --- | --- |
| Kankoso 301 | 0.005 |
| Swertia herb extract | 3 |
| Dipotassium glycyrrhizinate | 0.1 |
| Hydrous crystalline $\alpha,\alpha$-trehalose | 0.03 |
| "$\alpha$-GLUCOSYL HESPERIDIN", a product name of glucosyl hesperidin commercialized by Hayashibara Biochemical Laboratories, Inc., Okayama, Japan | 0.01 |
| Extract powder of indigo plant prepared by the method in Example 1 | 0.15 |
| Seaweed extract | 0.75 |
| Glycerin | 2 |
| Ethanol | 45 |
| Refined water | an amount sufficient to adjust the total amount to 100% |

Since the product is a hair tonic which has an effect of preventing both inflammation in the scalp and ageing and has a relatively strong antibacterial activity, it exerts an improved hair growth effect, prevents dandruff and urtication, inhibits loss of hair, and keeps the scalp clean. When applied to the scalp implanted with hair roots, the product promotes the growth of papilla pili cells and exerts a satisfactory effect on the improvement of survival rate of the hair roots implanted in the scalp.

Example 14

<Hair tonic>

A hair tonic was prepared in usual manner based on the following prescription.

| <Prescription> | (%) |
| --- | --- |
| Kankoso 301 | 0.005 |
| 1,3-Butylene glycol | 5 |
| Concentrated glycerin | 2 |
| "TORNARE®", a product name of a saccharide containing saccharide derivatives of $\alpha,\alpha$-trehalose commercialized by Hayashibara Biochemical Laboratories, inc., Okayama, Japan | 0.5 |
| Ascorbic acid 2-glucoside | 1 |
| "$\alpha$G RUTIN", a product name of glycosyl rutin commercialized by Hayashibara Biochemical Laboratories, Inc., Okayama, Japan | 0.001 |
| "$\alpha$-GLUCOSXL HESPERIDIN", a product name of glucosyl hesperidin commercialized by Hayashibara Biochemical Laboratories, Inc., Okayama, Japan | 0.05 |
| Extract powder of indigo plant prepared by the method in Example 1 | 0.1 |
| Arginine | 0.7 |
| $\ell$-Menthol | 0.2 |
| Ethanol | 40 |
| Refined water | an amount sufficient to adjust the total amount to 100% |

Since the product is a hair tonic which has an effect of preventing both inflammation in the scalp and ageing and has a relatively strong antibacterial activity, it exerts an improved hair growth effect, prevents dandruff and urtication, inhibits loss of hair, and keeps the scalp clean. When applied to the scalp implanted with hair roots, the product promotes the growth of papilla pili cells and exerts a satisfactory effect on the improvement of survival rate of the hair roots implanted in the scalp.

Example 15

<Soap>

To 96.5 parts by weight of a neat soap, obtained by subjecting a mixture of tallow and palm oil (=4:1 by weight) to conventional saponification and salting out method, were added one part by weight of an extract powder of indigo plant prepared in accordance with the method in Example 3, 1.5 parts by weight of a spray-dried product of "HALLODEX®", a product name of a saccharide containing saccharide derivatives of $\alpha,\alpha$-trehalose commercialized by Hayashibara Biochemical Laboratories Inc., Okayama, Japan, 0.5 part by weight of "AA2G", a product name of L-ascorbic acid 2-glucoside commercialized by Hayashibara Biochemical Laboratories Inc., Okayama, Japan, 0.5 part by weight of white sugar, 0.5 part by weight of "$\alpha$G RUTIN", a glycosyl rutin commercialized by Hayashibara Biochemical Laboratories Inc., Okayama, Japan, one part by weight of maltitol, 0.0001 part by weight of Kankoso 201, and an adequate amount of a flavor. The mixture was mixed to homogeneity, poured into a mold, cooled, and solidifies to obtain a soap. The product has an improved skin-whitening effect and slimming effect by L-ascorbic acid 2-glucoside, does not cause the skin dry after use, and has a satisfactory feeling in use. Also the product has an improved anti-ageing effect.

Example 16

<Toothpaste>

A toothpaste was prepared in usual manner based on the following prescription.

| <Prescription> | (%) |
|---|---|
| Calcium secondary phosphate | 45 |
| Pullulan | 2.9 |
| Sodium lauryl sulfate | 1.5 |
| Glycerin | 20 |
| Polyoxyethylene sorbitan laurate | 0.5 |
| Sorbitol | 10 |
| Maltitol | 7 |
| Extract powder of indigo plant prepared by the method in Example 1 | 0.5 |
| Tetrahydrocurcumin | 0.5 |
| Antiseptic | q.s. |
| Refined water | an amount sufficient to adjust the total amount to 100% |

Since the product contains the extract powder of indigo plant, it retains the desired alveolar strength and elasticity, has an improved anti-inflammatory effect, and has usefulness as cosmetics to retain and promote the health of oral cavity. The antibacterial ingredients, contained in the indigo extract, exert bacteriostat and bactericidal effects on microorganisms as causatives of dental caries and periodontal diseases existing on the surface of teeth and in periodontal pockets, and thus it can be advantageously used to prevent dental caries and to inhibit the onset and progress of periodontal diseases.

Example 17

<Toothpaste>

A toothpaste was prepared in usual manner based on the following prescription.

| <Prescription> | (%) |
|---|---|
| β-Glycyrrhetinic acid | 0.05 |
| Cetylpyridinium chloride | 0.05 |
| Calcium phosphate, dibasic | 29 |
| Extract powder of indigo plant prepared by the method in Example 2 | 2 |
| Concentrated glycerin | 20 |
| "TORNARE®" a product name of a saccharide containing saccharide derivatives of $\alpha,\alpha$-trehalose commercialized by Hayashibara Biochemical Laboratories, inc., Okayama, Japan | 10 |
| Silicic acid anhydride | 5 |
| Titanium oxide | 2 |
| Sodium lauryl sulfate | 1.2 |
| Sodium carboxymethylcellulose | 1.2 |
| Polyoxyethylene hydrogenated castor oil | 1 |
| Ethanol | 0.5 |
| Propolis extract | 0.5 |
| Magnesium phosphate | 0.3 |
| Sodium N-lauroyl sarcosinate | 0.2 |
| Maltitol | 0.1 |
| p-Hydroxybenzoate ester | 0.1 |
| Flavor | q.s. |
| Refined water | an amount sufficient to adjust the total amount to 100% |

Since the product contains the extract powder of indigo plant, it retains the desired alveolar strength and elasticity, has an improved anti-inflammatory effect, and has usefulness as a toothpaste to retain and promote the health of oral cavity. The antibacterial ingredients, contained in the indigo extract, exert bacteriostat and bactericidal effects on microorganisms as causatives of dental caries and periodontal diseases existing on the surface of teeth and in periodontal pockets, and thus it can be advantageously used to prevent dental caries and to inhibit the onset and progress of periodontal diseases.

Example 18

<Gel-type dentifrice>

A gel-type dentifrice was prepared in usual manner based on the following prescription.

| <Prescription> | (%) |
|---|---|
| β-Glycyrrhetinic acid | 0.05 |
| Cetylpyridinium chloride | 0.05 |
| Sorbitol solution | 30 |
| Extract powder of indigo plant prepared by the method in Example 2 | 1 |
| Concentrated glycerin | 10 |
| "TORNARE®", a product name of a saccharide containing saccharide derivatives of $\alpha,\alpha$-trehalose commercialized by Hayashibara Biochemical Laboratories, Inc., Okayama, Japan | 10 |
| Silicic acid hydride | 9 |
| Silicic acid anhydride | 7 |
| Xylitol | 3 |
| Sodium carboxymethylcellulose | 1 |

(continued)

| <Prescription> | (%) |
|---|---|
| "α-GLUCOSYL HESPERIDIN", a product name of glucosyl hesperidin commercialized by Hayashibara Biochemical Laboratories, Inc., Okayama, Japan | 1 |
| Polyoxyethylene hydrogenated castor oil | 0.5 |
| Ethanol | 0.5 |
| Propolis extract, commercialized by Hayashibara Biochemical Laboratories Inc., Okayama, Japan | 0.5 |
| Maltitol | 0.1 |
| p-Hydroxybenzoate ester | 0.1 |
| Flavor | q.s. |
| Refined water | an amount sufficient to adjust the total amount to 100% |

Since the product contains the extract powder of indigo plant, it retains the desired alveolar strength and elasticity, has an improved anti-inflammatory effect, and has usefulness as a dentifrice capable of retaining and promoting the health of oral cavity. The antibacterial ingredients, contained in the indigo extract, exert bacteriostat and bactericidal effects on microorganisms as causatives of dental caries and periodontal diseases existing on the surface of teeth and in periodontal pockets, and thus it can be advantageously used to prevent dental caries and to inhibit the onset and progress of periodontal diseases.

When the product was administered to 10 patients suffering from periodontal diseases two times a day every in the morning and evening, for 30 days, eight patients among the 10 were improved or even diminished their intraoral inflammatory and swelling in their gums, and six patients among the 10 were improved or even diminished their bleeding during brushing or when heavily pressing their gums.

Example 19

<Mouthwash>

A mouthwash was prepared in usual manner based on the following prescription.

| <Prescription> | (%) |
|---|---|
| Ethanol | 15 |
| "HALLODEX®", a syrup containing saccharide derivatives of α,α-trehalose, commercialized by Hayashibara Shoji Inc., Okayama, Japan | 8 |
| Extract powder of indigo plant prepared by the method in Example 3 | 0.5 |
| "α-GLUCOSYL HESPERIDIN", a product name of glucosyl hesperidin commercialized by Hayashibara Biochemical Laboratories, Inc., Okayama, Japan | 1 |
| Polyoxyethylene hydrogenated castor oil | 2 |
| Saccharine sodium | 0.02 |
| Sodium benzoate | 0.05 |
| Calcium phosphate, dibasic | 0.1 |
| Flavor | q.s. |
| Water | 71.7 (an amount sufficient to adjust the total amount to 100%) |

Since the product contains the extract powder of indigo plant, it retains a desired alveolar strength and elasticity, improves dry mouth induced by Sjögren syndrome, etc., prevents and treats intraoral inflammatory and dysgeusia, and has a satisfactory feeling in use as a mouthwash. The antibacterial ingredients, contained in the indigo extract, exert bacteriostat and bactericidal effects on microorganisms as causatives of dental caries and periodontal diseases existing on the surface of teeth and in periodontal pockets, and thus it can be advantageously used to prevent dental caries and to inhibit the onset and progress of periodontal diseases.

When applied to 11 patients suffering from stomatitis two times a day every in the morning and evening for seven days, nine patients among the 10 were improved or diminished their stomatitis.

Example 20

<Ointment>

An ointment was prepared in usual manner based on the following prescription.

| <Prescription> | (%) |
| --- | --- |
| Sodium acetate | 1 |
| Calcium | 4 |
| Glycerin | 10 |
| Peppermint oil | 0.5 |
| Extract powder of indigo plant prepared by the method in Example 4 | 0.6 |
| L-Ascorbic acid 2-glucoside | 2 |
| Petrolatum | 49 |
| Vegetable wax | 10 |
| Lanolin | 10 |
| Sesame oil | 10.5 |

The product is useful as an external dermal agent for skin-whitening and/or skin-beautifying. Also the product has an improved permeability to and extensibility over the skin, and it can be used for the purpose of maintaining/promoting the skin health.

Example 21

<Gel ointment>

A gel ointment was prepared in usual manner based on the following prescription.

| <Prescription> | (%) |
| --- | --- |
| Glycerin | 44.49 |
| Ethanol | 30 |
| Extract powder of indigo plant prepared by the method in Example 5 | 0.4 |
| Bittern solution (hardness 54,000 mg/ml) | 5 |
| "HIVISWAKO 104", a gel base | 1.5 |
| Polyoxyethylene (20) sorbitan monooleate | 1.5 |
| Kankoso 201 | 0.01 |
| 2,2',2"-Nitrotriethanol | 2.5 |

The product is useful as an external dermal agent for skin-whitening and/or skin-beautifying. Also the product has an improved permeability to and extensibility over the skin, and it can be used for the purpose of maintaining/promoting the skin health.

Example 22

<Bath salt>

A bath salt was prepared in usual manner based on the following prescription.

| <Prescription> | (%) |
|---|---|
| Hydrous crystalline $\alpha,\alpha$-trehalose, a product for cosmetic use, commercialized by Hayashibara Biochemical Laboratories Inc., Okayama, Japan | 74.4 |
| Sodium hydrogencarbonate | 12.5 |
| Extract powder of indigo plant prepared by the method in Example 1 | 2.5 |
| A powder containing $\alpha,\alpha$-trehalose and bittern in an amount with a weight ratio of 144:202, prepared by the method in Example 9 disclosed in International Patent Kokai No. WO 2003/016325 | 5.5 |
| "$\alpha$-GLUCOSYL HESPERIDIN", a product name of glucosyl hesperidin commercialized by Hayashibara Biochemical Laboratories, Inc., Okayama, Japan | 3 |
| Kankoso 201 | 0.0015 |
| Flavor | q.s. |
| Pigment | q.s. |
| | (an amount sufficient to adjust the total amount to 100%) |

The product is a bath salt with an improved slimming effect, skin-whitening effect, and/or skin-beautifying effect.

Example 23

<Cachou Film>

A cachou film was prepared in usual manner based on the following prescription.

| <Prescription> | (%) |
|---|---|
| "PULLULAN", a food additive of pullulan commercialized by Hayashibara Shoji Inc., Okayama, Japan | 22 |
| Carrageenan | 1 |
| Xanthan gum | 0.15 |
| Locust bean gum | 0.15 |
| Maltitol | 0.8 |
| Deionized water | 69.25 |
| Extract powder of indigo plant prepared by the method in Example 1 | 3 |
| Emulsified mint oil | 2.6 |
| Propolis extract | 0.5 |
| Sucralose | 0.3 |
| Citric acid | 0.25 |

The product retains the desired alveolar strength and elasticity and has an improved anti-inflammatory effect because it contains the extract powder of indigo plant, and thus it can be used for the purpose of maintaining/promoting oral health, with a commercial index of the above-identified effect. Also the product is a cachou film useful for preventing mouth smell.

Example 24

<Chewing gum>

Forty-five parts by weight of a gum base, 54.7 parts by weight of sucrose, 10 parts by weight of an extract powder of indigo plant prepared by the method in Example 4, and one part by weight of "HAYASHIBARA'S HESPERIDIN S", a product name of a glucosyl hesperidin commercialized by Hayashibara Shoji Inc., Okayama, Japan, were admixed with adequate amounts of a flavor and a pigment to adjust the total amount to 100% and processed into a chewing gum.
The product retains a desired alveolar strength and elasticity because it contains the extract powder of indigo plant and has an improved anti-inflammatory effect, and thus it can be used for the purpose of maintaining/promoting the skin-whitening, skin-beautifying, and oral health, as well as preventing/alleviating obesity, with a commercial index of the above-identified effect.

Example 25

<Candy>

Ninety-five parts by weight of "POWDERED MABIT", a product name of a maltitol-containing powder with a maltitol content of 93.5% by weight, d.s.b., commercialized by Hayashibara Shoji Inc., Okayama, Japan, and seven parts by weight of "HALLODEX®", a saccharide composition containing α,α-trehalose, commercialized by Hayashibara Shoji Inc., Okayama, Japan, were admixed with 32 parts by weight of water, dissolved by heating, and concentrated up to give a temperature of about 160° C under a reduced pressure. Just after completion of the concentration, the resulting mixture was admixed with 0.1 part by weight of a mint extract, 0.1 part by weight of a nandin extract, 0.1 part by weight of a chinese quince extract, and two parts by weight of an extract powder of indigo plant prepared by the method in Example 1 were mixed together. The concentrated solution thus obtained was formed into granules, three grams each, in a deposit manner to obtain a hard candy with a moisture content of about 1.8% by weight.

Since the product has an anti-inflammatory action, alleviates inflammatory symptoms of epithelial cells such as respiratory organs including intraoral tissues, throat, and trachea, as well as inflammation inherent to causatives of periodontal diseases or such diseases, and has an action of protecting intraoral or its adjacent cells and tissues, it can be advantageously used as a candy for the purpose of alleviating respiratory diseases such as cold syndrome and allergic diseases and of reducing throat or intraoral pains and unpleasantness due to air pollution, exhausted gases, tobaccos' smoke, etc., as well as of preventing periodontal diseases and ameliorating such symptom. Also the product can be used for the purpose of skin-whitening and skin-beautifying or preventing/alleviating obesity.

When the product was administered to 15 patients suffering from periodontal diseases three times a day every in the morning, at noon, and in the evening at a dose of two tablets in each administration, and allowed to melt intraorally, for successive 60 days, 10 patients among the 15 improved or even diminished their intraoral inflammation and swelling of gums, and eight patients among the 15 improved or even diminished their bleeding during brushing or when heavily pressing their gums.

Example 26

<Gummy candy>

To 15.6 parts by weight of water was added four parts by weight of collagen before mixing, and the mixture was admixed with 7.7 parts by weight of gelatin, followed by swelling the gelatin while reducing the pressure and keeping the condition for two to three minutes. Thereafter, when the temperature of the content became 65° C by heating under a reduced pressure, the heating was suspended to obtain a gelatin solution. A saccharide solution was prepared by mixing 21.7 parts by weight of sucrose, 7.3 parts by weight of trehalose, 7.3 parts by weight of "PO-300", a product name of a hydrogenated starch saccharified product commercialized by TOWA-KASEI Co., Ltd., Tokyo, Japan, 21.7 parts by weight of anhydrous crystalline glucose, and 10.9 parts by weight of water under stirring conditions; heating the mixture to 120° C: and lowering the heated mixture to 80° C by a reduced-pressure-concentration treatment to concentrate the resulting saccharide solution up to give an amount of 59.5 parts by weight. To 59.5 parts by weight of which, kept at about 65° C, was added and mixed with 27.3 parts by weight of the gelatin solution, preheated to about 65° C, and the resulting mixture was admixed with 2.5 parts by weight of anhydrous citric acid, 2.5 parts by weight of a lemon juice, 3.9 parts by weight of hardly assimilable dextrin, two parts by weight of an extract powder of indigo plant prepared by the method in Example 2, 2.4 parts by weight of water, and 0.9 part by weight of a flavor, and mixed to homogeneity by stirring to obtain a solution for candy (brix 74). Thereafter, the solution was placed into an injection apparatus, injected into molds made of corn starch from the injection nozzle tip in an amount of about one gram per mold, treated with drying at a temperature of 20° C and a humidity of 50% for 90 hours, and removed from the molds to obtain a gummy candy. The gummy candy thus obtained had a moisture content of about 12%.

Since the product contains the extract powder of indigo plant, it has an anti-inflammatory/antiseptic action, and thus it alleviates cytotoxicity against epithelial cells such as respiratory organs including intraoral tissue, throat, and trachea, as well as inflammatory symptoms associated with the cytotoxicity and inflammation inherent to causatives of periodontal diseases or such diseases, and has an action of protecting intraoral or its adjacent cells and tissues, it can be advantageously used as a candy for the purpose of alleviating respiratory diseases such as cold syndrome and allergic diseases and of reducing throat or intraoral pains and unpleasantness due to air pollution, exhausted gases, tobaccos' smoke, etc., as well as of preventing periodontal diseases and ameliorating such symptom. Also the product can be used for the purpose of skin-whitening and skin-beautifying or preventing/alleviating obesity.

Example 27

<Chocolate>

Using a chocolate, incorporated with 20 parts by weight of cacao mass as a chocolate dough, 22 parts by weight of whole milk powder, 16 parts by weight of cocoa butter, 42 parts by weight of sugar, three parts by weight of an extract powder of indigo plant prepared by the method in Example 1, and 0.1 part by weight of a flavor, it was subjected to conching for 23 hours, admixed with two parts by weight of yolk lecithin against 98 parts by weight of the resulting chocolate, followed by dispersing to homogeneity with the conching machine for another one hour. Thereafter, the resultant was subjected to tempering and molded. The product is a chocolate that satisfactory melts in the mouth and has a refreshing taste without lasting taste in the mouth. Since the product has an anti-inflammatory/antiseptic action, alleviates any cytotoxicity against epithelial cells such as respiratory organs including intraoral tissue, throat, and trachea, as well as inflammatory symptoms associated with the cytotoxicity and inflammation inherent to causatives of periodontal diseases or such diseases, and has an action of protecting intraoral or its adjacent cells and tissues, it can be advantageously used for healing throat for the purpose of alleviating respiratory diseases such as cold syndrome and allergic diseases and of reducing throat or intraoral pains and unpleasantness due to air pollution, exhausted gases, tobaccos' smoke, etc., as well as of preventing periodontal diseases and ameliorating such symptom. Also the product can be used for the purpose of skin-whitening and skin-beautifying or preventing/alleviating obesity.

Example 28

<Dietary supplement>

Five parts by weight of coenzyme $Q_{10}$, 26 parts by weight of "HAYASHIBARA ROYAL JELLY EXTRACT X", a royal jelly extract commercialized by Hayashibara Shoji Inc., Okayama, Japan, five parts by weight of a powdered sugar, 50 parts by weight of erythritol, eight parts by weight of L-ascorbic acid 2-glucoside, one part by weight of vitamin $B_1$, one part by weight of vitamin $B_2$, one part by weight of vitamin $B_6$, two parts by weight of extract powder of indigo plant prepared by the method in Example 1, one part by weight of "HAYASHIBARA'S HESPERIDIN S", a product name of a glucosyl hesperidin commercialized by Hayashibara Shoji Inc., Okayama, Japan, and one part by weight of a flavor were mixed and subjected to extrusion granulation and fluidized-bed drying to prepare a granular dietary supplement. Since the product has usefulness for maintaining the health, has an anti-inflammatory/antiseptic action, alleviates cytotoxicity against epithelial cells such as respiratory organs including intraoral tissue, throat, and trachea, as well as inflammation inherent to causatives of periodontal diseases or such diseases, and has an action of protecting intraoral or its adjacent cells and tissues, it can be advantageously used for alleviating respiratory diseases such as cold syndrome and allergic diseases, healing throat and intraoral pains and unpleasantness due to air pollution, exhausted gases, tobaccos' smoke, etc., as well as for preventing periodontal diseases and ameliorating their symptoms. Also the product can be used for the purpose of skin-whitening and skin-beautifying, controlling lipid metabolism, or preventing/alleviating obesity.

Industrial Applicability

As described above the extract powder of indigo plant of the present invention has a relatively low hygroscopicity, satisfactory fluidability, and improved storage stability. Also the extract powder has various physiological activities such as melanin formation inhibition, elastase inhibitory activity, lipase inhibitory activity, secretion regulatory action on sebum secreted from sebaceous crypt, SOD-like action, and DPPH radical-entrapping action, and thus the external dermal agents and orally ingestible compositions, which are incorporated with the extract powder, have an improved hypochromic effect and skin-whitening effect on pigmentation after sunburn, spots, freckles, and chloasma, and recover/maintain the strength/elasticity of the skin based on the elastase inhibitory activity, resulting in preventing ageing of the skin and maintaining the desired youthful skin conditions. In addition to the above actions, when used in combination with other skin-whitening ingredients, the compositions incorporated with the extract powder of indigo plant of the present invention can be augmented their effects and thus they can be used not only for the purpose of skin-whitening and skin-beautifying but for the prevention of oxidization of sebum ingredients in the skin, oxidative damage in the skin, and skin ageing; the protection of the skin; the treatment of dermatophytosis, stomatitis, and acne; the prevention and treatment of periodontal diseases and dental caries; and the reduction of body fats. The above compositions can be successively used safely and comfortably for a relatively long period of time without fear of causing side effects. The present invention is a significant invention that has the above-identified distinct effects and functions and greatly contributes to this art.

**Claims**

1. A process for producing an extract powder of indigo plant, **characterized in that** it comprises the steps of:

   incorporating a partial starch hydrolyzate into one part by weight, on a dry solid basis, of an indigo plant extract contained in a liquid extract of indigo plant in an amount of not lower than 0.25 part by weight but not higher than 5 parts by weight, on a dry solid basis; freeze-drying or spray-drying the mixture; and optionally pulverizing the resulting mixture.

2. The process of claim 1, **characterized in that** wherein said partial starch hydrolyzate is a dextrin and/or a non-reducing oligosaccharide with a dextrose equivalent (DE) of not lower than 0.2 but not higher than 10.2.

3. The process of claim 1 or 2, **characterized in that** wherein the indigo plant used for said extract powder of indigo plant is a plant of *Polygonum tinctorium* Lour.

4. An extract powder of indigo plant, whenever prepared by the process of any one of claims 1 to 3.

5. A process of a composition, **characterized in that** it comprises a step of incorporating said extract powder of indigo plant of claim 4 in an amount of 0.0005 to 30% in terms of indigo plant extract, on a dry solid basis.

6. The process of claim 5, wherein said composition is any one of cosmetics, quasi-drugs, pharmaceuticals, food products, feeds, baits, pet foods, and miscellaneous goods.

7. The process of claim 6, wherein said cosmetics, quasi-drugs, and pharmaceuticals are external dermal agents.

8. The process of claim 7, which contains a step of incorporating another ingredient(s) with skin-whitening effect in combination with said extract powder of indigo plant.

9. Use of said extract powder of indigo plant of claim 4 as a slimming agent.

10. Use of said extract powder of indigo plant of claim 4 as a skin-whitening agent.

11. Use of said extract powder of indigo plant of claim 4 as an agent for treating and/or preventing periodontal diseases.

12. Use of said extract powder of indigo plant of claim 4 as an anti-inflammatory.

FIG. 1

FIG. 2

FIG. 3

FIG. 4

**INTERNATIONAL SEARCH REPORT**

| International application No. |
|---|
| PCT/JP2007/072628 |

A.  CLASSIFICATION OF SUBJECT MATTER
*A61K36/70*(2006.01)i, *A23K1/16*(2006.01)i, *A23L1/30*(2006.01)i, *A61K8/73*
(2006.01)i, *A61K8/97*(2006.01)i, *A61K47/36*(2006.01)i, *A61P1/00*(2006.01)i,
*A61P1/02*(2006.01)i, *A61P1/16*(2006.01)i, *A61P1/18*(2006.01)i,
According to International Patent Classification (IPC) or to both national classification and IPC

B.  FIELDS SEARCHED

Minimum documentation searched (classification system followed by classification symbols)
A61K36/70, A23K1/16, A23L1/30, A61K8/73, A61K8/97, A61K47/36, A61P1/00,
A61P1/02, A61P1/16, A61P1/18, A61P3/04, A61P3/06, A61P3/10, A61P9/00,
A61P9/10, A61P11/00, A61P13/12, A61P17/00, A61P17/14, A61P17/16, A61P17/18,

Documentation searched other than minimum documentation to the extent that such documents are included in the fields searched
    Jitsuyo Shinan Koho      1922-1996   Jitsuyo Shinan Toroku Koho   1996-2008
    Kokai Jitsuyo Shinan Koho   1971-2008   Toroku Jitsuyo Shinan Koho   1994-2008

Electronic data base consulted during the international search (name of data base and, where practicable, search terms used)
    BIOSIS/MEDLINE/WPIDS(STN), CAplus(STN), JMEDPlus(JDream2),
    JSTPlus(JDream2)

C.  DOCUMENTS CONSIDERED TO BE RELEVANT

| Category* | Citation of document, with indication, where appropriate, of the relevant passages | Relevant to claim No. |
|---|---|---|
| Y | JP 60-2244465 A   (General Foods Corp.),<br>08 November, 1985 (08.11.85),<br>& JP 6-22453 B          & US 4582716 A<br>& EP 155848 A          & ES 541362 A<br>& CA 1220933 A          & IE 56014 A<br>& IN 162856 A          & AT 35501 A<br>& KR 9208855 A | 1-12 |
| Y | JP 48-10217 B   (Kon Purodakutsu Co.),<br>02 April, 1973 (02.04.73),<br>& GB 1240557 A          & FR 2013613 A<br>& CA 928136 A          & DE 1937687 B<br>& SE 361407 B          & ES 369804 A<br>& NL 691187 A          & BE 736345 A | 1-12 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    04 February, 2008 (04.02.08) | Date of mailing of the international search report<br>    12 February, 2008 (12.02.08) |
|---|---|
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2007/072628 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 3-228568 A  (Yoshihide HAGIWARA),<br>15 August, 1991 (15.08.91),<br>Example 1<br>(Family: none) | 1-12 |
| Y | Cai, Y. Z. et al, Production and properties of spray-dried Amaranthus betacyanin pigments, Journal of Food Science, 2000. Vol.65, No.7, pp.1248-1252 | 1-12 |
| Y | JP 2005-179216 A  (Kabushiki Kaisha Nomura),<br>07 July, 2005 (07.07.05),<br>Par. Nos. [0013], [0031]; table 3<br>(Family: none) | 6-8 |
| Y | Shin'ichiro INOUE et al., "Koshiboshoku Yudosei Koshikessho ni Oyobosu Ryoran (Polygonum tinctorium Lour.) no Kaizen Koka", Nat. Med., 2000, Vol.54, No.5, pages 261 to 264, Fig.2 | 6,7,9 |
| Y | IWAKI Kanso et al, Antimicrobial activity of Polygonum tinctorium Lour: extract against oral pathogenic bacteria, J. Nat. Med., 2006, Vol.60, No.2, Page.121-125 | 6,7,11 |
| Y | Masataka SUNAGAWA et al., "Taderan no Shishu Chiryo eno Oyo", Journal of Japan Dental Society of Oriental Medicine, 31 August, 2006 (31.08.06), Vol.25, No.1-2, pages 7 to 12 | 6,7,11 |
| Y | JP 2006-241080 A  (Hirosaki University),<br>14 September, 2006 (14.09.06),<br>Claims 2, 3<br>(Family: none) | 6,7,12 |
| Y | Takahiro SUNAYAMA et al., "Taderan no Koensho Sayo Candidasis Chiryo Hokoku", Medicine and Biology, 1999, Vol.139, No.1, pages 29 to 32 | 6,7,12 |

Form PCT/ISA/210 (continuation of second sheet) (April 2007)

| **INTERNATIONAL SEARCH REPORT** | International application No. |
|---|---|
| | PCT/JP2007/072628 |

Continuation of A. CLASSIFICATION OF SUBJECT MATTER
  (International Patent Classification (IPC))

*A61P3/04*(2006.01)i, *A61P3/06*(2006.01)i, *A61P3/10*(2006.01)i,
*A61P9/00*(2006.01)i, *A61P9/10*(2006.01)i, *A61P11/00*(2006.01)i,
*A61P13/12*(2006.01)i, *A61P17/00*(2006.01)i, *A61P17/14*(2006.01)i,
*A61P17/16*(2006.01)i, *A61P17/18*(2006.01)i, *A61P29/00*(2006.01)i,
*A61P31/04*(2006.01)i, *A61P31/10*(2006.01)i, *A61P31/12*(2006.01)i,
*A61P35/00*(2006.01)i, *A61P37/06*(2006.01)i, *A61P37/08*(2006.01)i,
*A61P39/06*(2006.01)i, *A61P43/00*(2006.01)i, *A61Q11/00*(2006.01)i,
*A61Q19/00*(2006.01)i, *A61Q19/02*(2006.01)i, *A61Q19/06*(2006.01)i,
*A61Q19/08*(2006.01)i

         (According to International Patent Classification (IPC) or to both national
         classification and IPC)


Continuation of B. FIELDS SEARCHED
  Minimum documentation searched (International Patent Classification (IPC))

A61P29/00, A61P31/04, A61P31/10, A61P31/12, A61P35/00, A61P37/06,
A61P37/08, A61P39/06, A61P43/00, A61Q11/00, A61Q19/00, A61Q19/02,
A61Q19/06, A61Q19/08

         Minimum documentation searched (classification system followed by
         classification symbols)

Form PCT/ISA/210 (extra sheet) (April 2007)

**EP 2 087 903 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001031581 A
- JP 2004189732 A
- WO 2003035091 A
- WO 0210361 A
- JP 2005095148 A
- JP 2005017642 W

**Non-patent literature cited in the description**

- Saikin-Shinkin-Kensa. 1982, 524-529